(19) Europäisches Patentamt
European Patent Office
Office européen des brevets

(11) EP 2 627 627 B1

(12) EUROPEAN PATENT SPECIFICATION

(45) Date of publication and mention of the grant of the patent:
**09.08.2017 Bulletin 2017/32**

(21) Application number: **11772888.1**

(22) Date of filing: **14.10.2011**

(51) Int Cl.:
*C07C 213/00* (2006.01) *C07B 53/00* (2006.01)
*C07C 213/10* (2006.01) *C07C 315/06* (2006.01)
*C07D 265/18* (2006.01)

(86) International application number:
**PCT/EP2011/005164**

(87) International publication number:
**WO 2012/048887 (19.04.2012 Gazette 2012/16)**

(54) **PROCESS FOR THE SYNTHESIS OF CHIRAL PROPARGYLIC ALCOHOLS**

VERFAHREN ZUR SYNTHESE VON CHIRALEN PROPARGYLALKOHOLEN

PROCÉDÉ DE SYNTHÈSE DE D'ALCOOLS PROPARGYLIQUES CHIRAUX CYCLIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2010 US 393221 P**
**14.10.2010 EP 10013633**

(43) Date of publication of application:
**21.08.2013 Bulletin 2013/34**

(73) Proprietor: **Bristol-Myers Squibb Company**
**Princeton, NJ 08543 (US)**

(72) Inventors:
- **BRENNER, Meinrad**
  **3940 Steg (CH)**
- **CARREIRA, Erick, M.**
  **8083 Zürich (CH)**
- **CHINKOV, Nicka**
  **19236 Haifa (IL)**
- **LORENZI, Miriam**
  **3904 Naters (CH)**
- **WARM, Aleksander**
  **1974 Arbaz (CH)**
- **ZIMMERMANN, Lothar**
  **3900 Brigerbad (CH)**

(74) Representative: **Reitstötter Kinzebach**
**Patentanwälte**
**Sternwartstrasse 4**
**81679 München (DE)**

(56) References cited:
**WO-A1-98/51676**

- **LIU L ET AL: "Enantioselective alkynylation of aromatic ketones promoted by (S)-phenylalanine-derived beta-amino alcohol", TETRAHEDRON ASYMMETRY, PERGAMON PRESS LTD, OXFORD, GB, vol. 15, no. 23, 29 November 2004 (2004-11-29), pages 3757-3761, XP004662081, ISSN: 0957-4166, DOI: DOI:10.1016/J.TETASY.2004.10.014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 627 627 B1

## Description

The invention is directed to a process for the preparation of chiral propargylic alcohols, which are key intermediates for the preparation of pharmaceuticals and agrochemicals and as precursors for compounds in the material sciences.

Jiang et al. disclosed in Tetrahedron Lett. 2002, 43, 8323-8325 and J. Org. Chem. 2002, 67, 9449-9451 the reaction of acetylene derivatives with aldehydes and ketones in the presence of equimolar amounts of a Zn(II) compound to give several racemic propargylic alcohols. Chiral compounds are not mentioned at all.

WO-A-95/20389, WO-A-96/37457, WHO 98/30543 and WO 98/30540 disclose several processes for the production of chiral propargylic alcohols useful for the synthesis of pharmaceuticals. WO-A-98/51676 disclose a process wherein by addition of a first chiral and optionally a second additive in a zinc(II) mediated reaction the chiral product is obtained in high enantiomeric excess. The disadvantage of said process is the use of high amounts of expensive zinc catalysts and chiral compounds.

Liu et al. Tetrahedron Asymmetry, 2004, 15, 3757-3761 describe an enantioselective alkynylation of aromatic ketones mediated by diethylzinc in the presence of a protic chiral ligand.

A main task for the present invention was therefore to supply an alternative process for the production of chiral propargylic alcohol with high enantiomeric excess. A further problem was to reduce the amounts of catalyst and other components to be added during the reaction in order to facilitate the workup procedures of the product and to promote industrial production.

The problem is solved by the process of claim 1. The inventive process comprises the addition of a starting amount of the chiral product to the reaction as a chiral mediator, which allows to reduce the amount of further chiral auxiliaries. Presence of the chiral product from the beginning of the reaction has the advantageous side effect that the amount of the zinc(II) catalyst can be reduced compared to processes known in the art. Furthermore, the addition of the compound of formula I allows to dispense with chiral auxiliaries, while still the chiral product is formed in high enantiomeric excess (ee).

Claimed is a process for the preparation of chiral compounds of the formula

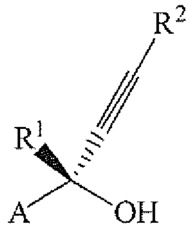

I,

or mirror image, wherein

$R^1$ is selected from the group consisting of hydrogen, $C_{1-6}$-alkyl and ($C_{1-6}$-alkoxy)carbonyl, any alkyl or alkoxy optionally being substituted with one or more halogen atoms,
$R^2$ is selected from the group consisting of aryl, aralkyl, $C_{1-6}$-alkyl and (1'-$R^3$)-$C_{3-6}$-cycloalkyl wherein $R^3$ is hydrogen, methyl or ethyl, and wherein any aryl, aralkyl, alkyl is optionally substituted with one or more halogen atoms, and
A is selected from the group consisting of $C_{1-20}$-alkyl, $C_{3-6}$-cycloalkyl, aryl and aralkyl, any cycloalkyl, aryl and aralkyl optionally being annulated to one or more further 5 to 7 membered carbocyclic or heterocyclic rings, and wherein any alkyl, cycloalkyl, aryl and aralkyl is optionally substituted with one or more substituents selected from halogen atoms, cyano, $C_{1-6}$-alkyl, $C_{3-6}$-cycloalkyl, $-NR^4R^5$, $-SR^6$, $S(O)R^6$ or $S(O_2)R^6$, and/or $-OR^7$, with $R^6$ is $C_{1-6}$-alkyl, optionally substituted with one or more halogen atoms,
$R^7$ is hydrogen or $C_{1-6}$-alkyl, optionally substituted with one or more halogen atoms, or, where

(a) $R^4$ and $R^5$ are independently selected from hydrogen or $C_{1-6}$-alkyl, or
(b) $R^4$ is hydrogen and $R^5$ is $C_{2-7}$-acyl or ($C_{1-6}$-alkoxy)carbonyl, wherein each acyl and alkoxy in $R^5$ in turn is optionally substituted with one or more halogen atoms, or
(c) $R^4$ and $R^5$ together with the nitrogen atom form a 5 to 7 membered heterocyclic ring, or
(d) $R^4$ and $R^5$ together are =CH-aryl, the aryl moiety optionally being substituted with one or more substituents selected from halogen atoms, $-NH_2$, $-NH(C_{1-6}$-alkyl), $-N(C_{1-6}$-alkyl$)_2$ or $C_{1-6}$-alkyl, or
(e) $R^4$ and $R^5$ together are $=CH-N(C_{1-6}$-alkyl$)_2$,

$R^6$ is $C_{1-6}$-alkyl, optionally substituted with one or more halogen atoms, and

$R^7$ is hydrogen or $C_{1-6}$-alkyl, optionally substituted with one or more halogen atoms, or, wherein A and $R^1$ together form a 5 to 7 membered carbocyclic or heterocyclic ring, optionally substituted with one or more substituents selected from halogen atoms, cyano, $C_{1-6}$-alkyl, $C_{3-6}$-cycloalkyl, $-NR^4R^5$, $-SR^6$, $S(O)R^6$ or $S(O_2)R^6$, and/or $-OR^7$, wherein $R^2$, $R^3$, $R^4$, $R^5$, $R^6$ and $R^7$ are as defined above, and wherein each alkyl and cycloalkyl substituent attached to A in turn is optionally substituted with one or more halogen atom,
said process comprising the steps of

(i) reacting a protic chiral auxiliary, wherein said protic chiral auxiliary is an ephedrine derivative selected from the group consisting of diastereoisomers of 2-(di-$C_{1-4}$-alkylamino)-1-phenyl-propan-1-ols, 2-(*N*,*N*-$C_{4-6}$-alkylene)-1-phenyl-propan-1-ols and 2-(1-hetcroaryl)-1-phenyl-propan-1-ols with a diorganylzinc(II) compound selected from the group consisting of di($C_{1-8}$-alkyl)zinc(II) and di($C_{3-6}$-cycloalkyl)zinc(II), in the presence of a solvent selected from the group consisting of tetrahydrofuran, benzene, chlorobenzene, *o*-, *m*-, *p*-dichlorobenzene, dichloromethane, toluene, *o*-, *m*-, and *p*-xylene, hexanes, heptanes, cyclohexane, pentane, 1,4-dioxane, cyclohexane, diethyl ether, *tert*-butyl methyl ether, diisopropyl ether, *N*-methylpyrrolidine, and mixtures thereof, at a temperature in the range of 0 to 40 °C, and
(ii) keeping the mixture of step (i), preferably under stirring, in a first maturation period until the reaction is completed, but of at least 20 min, preferably between about 20 to 120 min, and
(iii) reacting the mixture obtained after step (ii) with a compound of formula

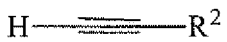

II,

wherein $R^2$ is as defined above,
(iv) keeping the mixture of step (iii), preferably under stirring, in a second maturation period until the reaction is completed, but of at least 10 min, preferably between about 10 to 120 min and
(v) reacting the mixture obtained after step (iv) with a compound of formula

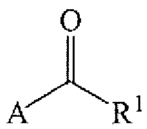

III,

wherein A and $R^1$ are as defined above, and an organolithium base selected from the group consisting of ($C_{1-6}$-alkyl)lithium, lithium diisopropylamide (LDA), lithium hexamethyldisilazide (LiHMDS), phenyllithiuzn, and naphthyllithium and/or another alkali metal compound selected from the group consisting of sodium or potassium $C_{1-6}$-alkoxides, sodium or potassium diisopropylamide, and sodium or potassium hexamethyldisilazide at a temperature in the range of 0 to 40 °C, and
(vi) keeping the mixture obtained in step (v) to 10 to 50 °C until the reaction is completed, to obtain the compound of formula I.

The major advantages of the present process are the reduction of the zinc(II) catalyst in view of the compound of formula III, the need of only one protic compound to first react with the zinc(II) catalyst, especially the possibility to avoid addition of fluorinated alcohols.

In contrast to known processes which require the addition of two different proton sources, wherein an additional proton source can be methanol, ethanol, propanol, isopropyl alcohol, butanol, isobutanol, *sec*-butanol, *tert*-butanol, pentanol, $(CH_3)_3CCH_2OH$, $(CH_3)_3CCH(CH_3)OH$, $Cl_3CCH_2OH$, $CF_3CH_2OH$, $CH_2{=}CHCH_2OH$, $(CH_3)_2NCH_2CH_2OH$ or even another chiral compound. The present process can be carried out with only one proton source, which at the same time acts as a chiral auxiliary. A preferred proton source in that sense is an ephedrine derivative, more preferably a 1-phenyl-2-(pyrrolidinyl)propan-1-ol derivative.

Here and hereinbelow the term "alkyl'" represents a linear or branched alkyl group. By using the form "$C_{1-n}$-alkyl" the alkyl group is meant having 1 to n carbon atoms. $C_{1-6}$-alkyl represents for example methyl, ethyl, propyl, isopropyl, butyl, isobutyl, *sec*-butyl, *tert*-butyl, pentyl and hexyl.

Herein the term "alkenyl" represents a linear or branched group carrying at least one carbon-carbon double bound. By using the form "$C_{2-n}$-alkenyl" is meant the main chain of the alkenyl group having 2 to n carbon atoms. $C_{2-6}$-alkenyl represents for example ethenyl (vinyl), propen-2-yl, propen-3-yl (allyl), buten-1-yl or hexen-1-yl.

Herein the term "alkynyl" represents a linear or branched group carrying at least one carbon-carbon triple bound. By using the form "$C_{2-n}$-alkynyl" is meant the main chain of the alkynyl group having 2 to n carbon atoms. $C_{2-6}$-alkynyl represents for example ethynyl, 1-propynyl, or 1-hexynyl.

Here and hereinbelow the term "dialkyl" independently means to alkyl groups attached to a connecting atom. For example in a dialkylzinc (II) compound, two alkyl groups are attached to zinc, whereas in dialkylamino the two alkyl groups are attached to nitrogen.

Here and hereinbelow the term "alkoxy" represents a linear or branched alkoxy group. By using the form "$C_{1-n}$-alkoxy" the alkyl group is meant having 1 to n carbon atoms. $C_{1-6}$-alkoxy represents for example methoxy, ethoxy, propoxy, isopropoxy, butoxy, isobutoxy, *sec*-butoxy, *tert*-butoxy, pentyloxy and hexyloxy.

Here and hereinbelow the term "cycloalkyl" represents a cycloaliphatic group having 3 carbon atoms or more. Cycloalkyl represents mono- and polycyclic ring systems, such as cyclopentyl, cyclohexyl, cycloheptyl, cyclooctyl, adamantyl or norbornyl.

Here and hereinbelow the term "aryl" represents an aromatic group, preferably phenyl or naphthyl.

Here and hereinbelow the term "heteroaryl" represents a heteroaromatic group, preferably pyridinyl, pyrimidinyl, furyl or thienyl.

Here and hereinbelow the term "aralkyl" represents a group having 7 or more carbon atoms, consisting of an alkyl and an aryl moiety, wherein the alkyl moiety of the aralkyl residue is a $C_{1-8}$ alkyl group and the aryl moiety is selected from the group consisting of phenyl, naphthyl, furanyl, thienyl, benzo[b]furanyl, benzo[b]thienyl.

The present process relies on a specific order of addition of the compounds of the diorganylzinc(II) compound, the compounds of formulae II and III comprising the two maturation periods of steps (ii) and (iv), respectively. The term "until the reaction is completed" in steps (ii), (iv) and (vi) means that at least 90% conversion, preferably at least 95%, more preferably 98%, is reached in the respective step. The course of conversion can be followed for example by calorimetric measurements, "React IR" or FT-IR. Also possible are off-line methods, such as gas chromatography or HPLC. It is possible to establish a correlation between conversion and the output of analytical methods easily with computer aided systems. We suspect that maybe in the first maturation period a first catalytic species if formed, while in the second maturation step a second catalytic species is formed. The first catalytic species might comprise a compound of formula (alkyl)Zn(chiral auxiliary) which might be solved in the mixture or aggregated. The second catalytic species might comprise a compound of formula (C=C-$R^2$)Zn(chiral auxiliary), wherein $R^2$ is as defined above. By using diethylzinc ethane evolution of approx. 1 equivalent in respect to diethylzinc could be observed in steps (ii) and (iv), respectively. Ethane formation could be detected during the diethylzinc addition. The ethane release was observed with a delay with respect to the diethylzinc addition. It is assumed that ethane was first dissolved in the reaction solution and then released to the gas phase. $^1$H-NMR analysis shows that some ethane remained dissolved in the reaction mixture. The structures of the catalytic species can be only proposed because of the difficulties to separate the catalytic species from the respective precursors. Especially, since catalytic species would be highly sensitive to air and humidity.

In step (v) the addition of the compound of formula III, and the organolithium base and/or the other alkali metal compound, are fed simultaneously, either separately or as a mixture. Advantageously, dosage of the organolithium base and/or the other alkali metal compound starts ahead of the dosage of the compound of formula III, preferably up to 20 min ahead, more preferably up to about 10 min ahead.

The process is designed to obtain the compound of formula I with an enantiomeric purity (*ep*) of at least 90%, preferably with an *ep* of at least 95%, more preferred of at least 96%, and even more preferred of at least 97%.

The protic chiral auxiliary induces the formation of the desired enantiomer during reaction of the compounds of formulae II and III. The expression "protic chiral auxiliary" means that the chiral auxiliary comprises at least one proton which can be easily removed, most preferred in a hydroxyl group.

In a preferred embodiment the chiral auxiliary is selected from protic *N*,*N*-disubstituted ephedrine derivatives.

Suitable protic *N*,*N*-disubstituted ephedrine derivatives are for example diastereoisomers of 2-(di-$C_{1-4}$-alkylamino)-1-phenyl-propan-1-ols, such as 2-(dimethylamino)-1-phenyl-propan-1-ol, 2-(diethylamino)-1-phenyl-propan-1-ol, 2-(diisopropylamino)-1-phenyl-propan-1-ol, and 2-(dibutylamino)-1-phenyl-propan-1-ol; 2-(*N*,*N*-$C_{4-6}$-alkylene)-1-phenyl-propan-1-ols, such as 1-phenyl-2-(piperidinyl)propan-1-ol and 1-phenyl-2-(pyrrolidinyl)propan-1-ol, and 2-(1-heteroaryl)-1-phenyl-propan-1-ols, such as 1-phenyl-2-(1-pyridinyl)propan-1-ol, 1-phenyl-2-(1-piridinyl)propan-1-ol. More specific examples are (1*R*,2*S*)-2-(dimethylamino)-1-phenyl-propan-1-ol (CAS [552-79-4]), (1*S*,2*R*)-2-(dimethylamino)-1-phenyl-propan-1-ol (CAS [42151-56-4]), (1*R*,2*R*)-2-(dimethylamino)-1-phenyl-propan-1-ol (CAS [14222-20-9]), (1*S*,2*S*)-2-(dimethyl-amino)-1-phenyl-propan-1-ol (CAS [51018-28-1]), (1*R*,2*S*)-1-phenyl-2-(pyrrolidinyl)propan-1-ol (CAS [127641-25-2]), (1*S*,2*R*)-1-phenyl-2-(pyrrolidinyl)propan-1-ol (CAS [123620-80-4]), (1*R*,2*R*)-1-phenyl-2-(pyrrolidinyl)propan-1-ol and (1*S*,2*S*)-1-phenyl-2-(pyrrolidinyl)propan-1-ol.

In a preferred embodiment the protic chiral auxiliary is (1*R*,2*S*)-1-phenyl-2-(pyrrolidinyl)propan-1-ol) to obtain ((*S*)-2-(2-amino-5-chlorophenyl)-4-cyclopropyl-1,1,1-trifluorobut-3-yn-2-ol (SD573) or one of its salts, from 1-(2-amino-5-chlorophenyl)-2,2,2-trifluoroethanone and cyclopropylacetylene.

The amount of the zinc(II) catalyst needed in the reaction can be reduced remarkably compared to processes

known in the art. It must be noted that the amount of the zinc(II) catalyst can be surprisingly much lower than the amount of the chiral auxiliary.

In a preferred embodiment the molar ratio of the protic chiral auxiliary to the diorganylzinc(II) compound is in the range of 1.5:1 to 1:1, preferably in the range of 1.3:1 to 1.2:1, most preferred at 1.24:1.

The chiral auxiliary mediates the catalytic process. Although one would expect that zinc(II) catalyst and the protic chiral auxiliary form a zinc(II) complex with a certain stoichiometry it is not necessary to add the chiral auxiliary and the zinc(II) catalyst in equimolar amounts. Preferably the amount of the chiral auxiliary is slightly higher than the amount of the diorganylzinc(II) catalyst.

Suitable diorganylzinc(II) compounds are for example selected from di($C_{1-8}$-alkyl) and di($C_{3-6}$-cycloalkyl), wherein the alkyl moieties are selected from the group consisting of methyl, ethyl, propyl, isopropyl, butyl, isobutyl, *sec*-butyl and *tert*-butyl, pentyl, hexyl, heptyl, and octyl, and wherein the cycloalkyl moieties are selected from the group consisting of cycloprapyl, cyclobutyl, cyclopentyl and cyclohexyl.

In a preferred embodiment in step (i) the molar ratio of the protic chiral auxiliary to the compound of formula III is in the range of 1:1 to 1:10, preferably in the range of 1:2 to 1:6, more preferably of 1:3 to 1:6.

Addition of the compound of formula III can be carried out at a temperature from 0 to +40 °C, preferably from +10 to +30 °C.

In a preferred embodiment the compound of formula II is selected from the group consisting of terminal $C_{3-8}$-alkylalkynes, cyclopropylacetylene, (1'-methyl)-cyclopropylacetylene and phenylacetylene.

It is recommended, that in step (iii) the compound of formula II is used in a molar ratio to the compound of formula III of 1:0.6 to 1:1.3

In a preferred embodiment the compounds of formula III are selected from the group consisting of *p*-methylbenzaldehyde, *p*-fluorobenzaldehyde, *p*-cyanobenzaldehyde, *p*-methoxybenzaldehyde, naphthalenealdehyde, cinnamaldehyde, $C_{3-20}$-alkane aldehydes, cyclohexyl carbaldehyde, cyclohexyl metyl ketone, methyl 4-metylcyclohexyl ketone, 1,1,1-trifluoroacetophenone and 2-(trifluoroaceto)-4-chloro-anilin.

In a further preferred embodiment the organolithium base and/or the other alkali metal compound is added in a molar ratio to the compound of formula III in the range of 1:0.8 to 1:1.5, preferably of 1:0.8 to 1:1.2.

A suitable organolithium base in the present process is selected from the group consisting of ($C_{1-6}$-alkyl)lithium, lithium diisopropylamide (LDA), lithium hexamethyldisilazide (LiHMDS), phenyllithium, and naphthyllithium.
Preferably the organolithium base is an organolithium compound or a lithium organic salt.

In preferred embodiment such organometallic lithium compound is selected from the group consisting of phenyllithium and $C_{1-6}$-alkyl)lithium.

Preferably said ($C_{1-6}$-alkyl)lithium is selected from the group consisting of methyllithium, *n*-butyllithium, *sec*-butyllithium, *tert*-butyllithium, and hexyllithium.

Preferably the other alkali metal compound is selected from sodium or potassium $C_{1-6}$-alkoxides, sodium or potassium diisopropylamide, and sodium or potassium hexamethyldisilazide.

The organolithium base and/or the other alkali metal compound can be used either independently or in mixtures of at least two different species.

During the addition of the organolithium base and/or the other alkali metal compound the reaction mixture is preferably kept at a temperature from +10 to +30 °C.

In the present process the solvent preferably is selected from the group consisting of aprotic nonpolar solvents, aprotic polar solvents and mixtures thereof.

The solvents of agents added in solution maybe selected independently of each other. Particularly preferred the solvent is selected from the group consisting of tetrahydrofuran, benzene, chlorobenzene, *o*-, *m*-, *p*-dichlorobenzene, dichloromethane, toluene, *o*-, *m*-, and *p*-xylene, hexanes, heptanes, cyclohexane, pentane, 1,4-dioxane, cyclohexane, diethyl ether, *tert*-butyl methyl ether, diisopropyl ether, *N*-methylpyrrolidine, and mixtures thereof.

Preferably by cyclisation the compound of formula I, wherein A is an optionally further substituted 2-aminophen-1-yl group can be used to obtain a compound of formula

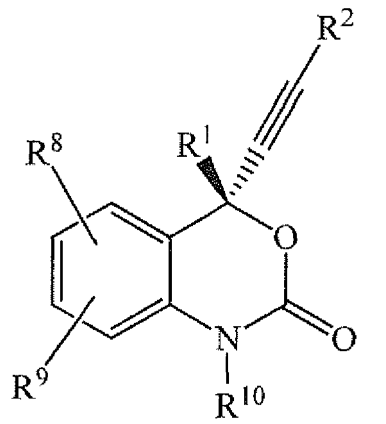

IV,

or mirror image, and/or a suitable salt thereof,
wherein

$R^1$ is selected from the group consisting of linear or branched $C_{1-6}$-alkyl or ($C_{1-6}$-alkoxy)-carbonyl, any alkyl or alkoxy optionally being substituted with one or more halogen atoms,
$R^2$ is selected from the group consisting of linear or branched $C_{1-6}$-alkyl, and $C_{3-6}$-cycloalkyl,
$R^8$ and $R^9$ are independently selected from the group consisting of hydrogen, halogen atom, and $C_{1-6}$-alkyl, optionally substituted with one or more halogen atoms, and
$R^{10}$ is hydrogen or a group selected from the group consisting of aryl, aralkyl, $C_{1-6}$-alkyl and ($C_{1-6}$-alkoxy)carbonyl, wherein the aryl moiety in any aryl or aralkyl is optionally substituted with one or more $C_{1-6}$-alkyl, $C_{1-6}$-alkoxy or $C_{3-8}$-cycloalkyl, each alkyl, alkoxy or cycloalkyl substituent is optionally substituted with one or more halogen atoms.

The chirality of the carbon atom in formulae I and IV which is attached to $R^1$, the $R^2$-alkynyl group and the hydroxy group is preferably maintained during cyclization.

**Examples:**

The chiral alkynylation reaction (Examples 1, 2, and 4) was performed two times with the respective starting compounds. Once using (1*R*,2*S*)-1-phenyl-2-(pyrrolidinyl)propan-1-ol as ligand and once using (1*S*,2*R*)-1-phenyl-2-(pyrrolidinyl)propan-1-ol as ligand. This allowed the unambiguous assignment of the two enantiomers of the products by HPLC. In the experimental details below, only the experiments using (1*S*,2*R*)-1-phenyl-2-(pyrrolidinyl)propan-1-ol are described in detail because there was no major difference between (1*R*,2*S*)-1-phenyl-2-(pyrrolidinyl)propan-1-ol and (1*S*,2*R*)-1-phenyl-2-(pyrrolidinyl)propan-1-ol. For the all examples using cyclopropylacetylene addition to a diethylzinc catalyst, an ethane release could be observed as well as described in Example 1. The configuration of the products of example 2 to 4 were tentatively assigned based on the assumption that reaction in presence of ligand (1*S*,2*R*)-1-phenyl-2-(pyrrolidinyl)propan-1-ol gives preferably the product with (*R*)-configuration in analogy to Example 1 (SD573 process), where the configuration of both enantiomers are well known. In the SD573 process (1*R*,2*S*)-1-phenyl-2-(pyrrolidinyl)propan-1-ol gives preferably the product with (*S*)-configuration. Procedures for analytical methods A to D are attached after the examples.

In all cyclisation examples, except where not expressively mentioned, the *ee* was not measured since in all cyclisation examples with final *ee*-measurement the product (for example DMP-266) always corresponded to the *ee* of the respective starting compound, for example in case of cyclisation of SD573-MSA or SD573 free base (CAS [209412-27-7], 99.6% *ee*) to DMP-266.

$$ee = \text{enantiomeric excess} = ((S)-(R))/((S)+(R))$$

$$ep = \text{enantiomeric purity} = (S)/((S)+(R))$$

**Example 1: (*S*)-2-(2-Amino-5-chlorophenyl)-4-cyclopropyl-1,1,1-trifluorobut-3-yn-2-ol mesylate (2:3 mol/mol) (SD573-MSA)**

A solution of (1*R*,2*S*)-1-phenyl-2-(pyrrolidinyl)propan-1-ol (18.1%-w/w, 171.6 g, 151 mmol)) in a THF/toluene mixture (9:1-w/w) was charged in a vessel and cooled to 17 °C. A solution of diethylzinc in toluene (29%-w/w, 52.0 g, 122 mmol) was added at 15 to 20 °C and the mixture was aged at said temperature for 30 min. Ethane (approx. 1 equivalent in respect to diethylzinc) was formed during the diethylzinc addition and partially released from the reaction mixture. The ethane release is observed with a delay with respect to the diethylzinc addition, since ethane is first dissolved in the reaction solution and then released to the gas phase. According to $^1$H-NMR analysis some ethane remained dissolved in the reaction mixture. A solution of cyclopropylacetylene (compound of formula II, wherein $R^2$ is cyclopropyl) in toluene (70%-w/w, 57.0 g, 600 mmol) was added at 15 to 20 °C and the resulting mixture was aged at 20 °C for 1 h. During the addition of cyclopropylacetylene additional ethane (approx. 1 equivalent in respect to diethylzinc) was formed and released to the gas phase. A solution of butyllithium (BuLi) in toluene (157.6 g, 2.92 mol/kg, 460 mmol) and a solution of 1-(2-amino-5-chlorophenyl)-2,2,2-trifluoroethanone (SD570, compound of formula III, wherein A is 2-amino-5-chlorophenyl and $R^1$ is trifluoromethyl) (40.1%-w/w, 278.0 g, 500 mmol) in THF/toluene (1:1-w/w) were added in parallel to the reaction mixture at 20 °C within 180 min. The addition of BuLi was started 10 min in advance of the SD570 addition. Butane was formed during BuLi addition. However, most of the butane remained dissolved in the reaction mixture and only weak gas formation was observed. The course of reaction can be followed online, for example by calorimetric measurements or by "React IR" also called "*in-situ* FTIR". After complete addition of SD570 the reaction mixture was stirred for 30 min at 20 °C, then heated to 30 °C over a period of 60 min and aged for 6 h at 30 °C. The reaction mixture was stirred at 0 °C overnight, diluted with toluene (218 g) at 20 °C and quenched by addition of aqueous citric acid (1 M, 375 g). After stirring for 15 min the phases were separated and the aqueous phase was discarded. The organic phase was successively washed with water (76 g), aqueous $NaHCO_3$ solution (5%-w/w, 200 g), and again water (100 g). The organic phase was partially concentrated, then diluted with toluene (250 g), again partially concentrated and diluted with toluene (976 g residue). The enantiomeric purity (*ep*) of (*S*)-2-(2-amino-5-chlorophenyl)-4-cyclopropyl-1,1,1-trifluorobut-3-yn-2-ol (SD573) in the crude product was approx. 96 to 97% according to Method B. Although not belonging to the preparation process, described is also a process to transfer the product in a more stable form as a methanesulfonic acid salt. The residue was diluted with isopropyl alcohol (126.6 g). Then Methanesulfonic acid (43.3 g) was added over a period of 30 min at 30 °C. Seeding crystals (between 1 and 10 mg) were added and the mixture aged for 30 min at 30 °C. A second portion of methanesulfonic acid (26.5 g) was added over a period of 60 min at 30 °C. The resulting solution was aged for 30 min at 30 °C and later cooled to 5 °C over a period of 60 min. After further aging at 5 °C for 30 min, the product was filtered and washed with cold toluene/isopropyl alcohol (10:1-w/w, 262 g) at 5 °C. The wet methanesulfonic acid salt of SD573 ((*S*)-2-(2-amino-5-chlorophenyl)-4-cyclopropyl-1,1,1-trifluorobut-3-yn-2-ol mesylate (2:3 mol/mol, SD573-MSA, compound of formula I, wherein A is 2-amino-5-chlorophenyl, $R^1$ is trifluoromethyl and $R^2$ is cyclopropyl) was dried *in vacuo* at 40 °C to obtain 188.3 g (432 mmol, 86.5% yield). SD573-MSA was obtained with a purity of 99.9% and 99.7% *ep*, according to Method A.

**Example 2: (*R*)-2-(2-Aminobiphenyl-3-yl)-4-cyclopropyl-1,1,1-trifluorobut-3-yn-2-ol methanesulfonate (1:1 mol/mol)**

(1*S*,2*R*)-1-phenyl-2-(pyrrolidinyl)propan-1-ol (20.3 g, 18.0 mmol) in THF/toluene (9:1-w/w, 18.2%-w/w) was charged under a nitrogen atmosphere to a dry, jacketed 150 mL-reactor with agitator. Diethylzinc in toluene (29.9%-w/w, 6.48 g, 15.7 mmol) was added by syringe keeping the temperature at 17 to 22 °C and the mixture was aged for 30 min at 17 °C. Cyclopropylacetylene in toluene (69.6%-w/w, 6.84 g, 72.0 mmol) was added at 17 °C and the resulting mixture was aged for about 60 min at 20 °C. To the reaction mixture BuLi in toluene (3.06 mol/kg, 19.9 g, 60.9 mmol) and (1-(4-aminobiphenyl-3-yl)-2,2,2-tiifluoroethanone (CN46225, compound of formula III, wherein A is 4-aminobiphenyl-3-yl and $R^1$ is trifluoromethyl) (43.0%-w/w, 37.0 g, 60 mmol) in THF/toluene (1:1-w/w) were added in parallel over a period of 3 h at 20 °C. The addition of BuLi was started about 10 min in advance of the CN46225 addition. After complete addition of BuLi and CN46225, the reaction mixture was stirred for 30 min at 20 °C, then heated over a period of 1 h to 30 °C and aged for 6 h at 30 °C. The reaction mixture was stirred overnight at 0 °C. HPLC indicated 94.3% conversion and 95.6% *ep*, according to Method B. The reaction mixture was diluted with toluene (27.6 g) at room temperature and quenched by adding aqueous citric acid (1 M, 45.3 g). The phases were separated and the aqueous phase was discarded. The organic phase was successively washed with water (9.1 g), aqueous $NaHCO_3$ (5%-w/w, 24.2 g) and water (12.0 g). The organic phase was heated under reduced pressure to partly remove THF while toluene is added to finally reach a THF poor residue (54.5 g) of (*R*)-2-(4-aminobiphenyl-3-yl)-4-cyclopropyl-1,1,1-trifluorobut-3-yn-2-ol (CN46630, compound of formula I, wherein A is 4-aminobiphenyl-3-yl, $R^1$ is trifluoromethyl and $R^2$ is cyclopropyl). The residue was diluted with isopropyl alcohol (16.7 g) and toluene (60.0 g). A first portion of methanesulfonic acid (5.48 g, 57.0 mmol) was added by a syringe pump over a period of 30 min at 30 °C. Seeding crystals (a small portion between 1 and 10 mg)

were added and the mixture was aged for 30 min at 30 °C. A second portion of methanesulfonic acid (2.88 g, 30.0 mmol) was added by syringe pump over a period of 45 min at 30 °C. The mixture was stepwise aged and cooled over 1 h 45 min to finally reach 5 °C. The product was filtered, and the filter cake was washed with toluene/isopropyl alcohol (10:1-w/w, 27.0 g) and *dried in vacuo* at 40 °C. The dry product (*R*)-2-(4-aminobiphenyl-3-yl)-4-cyclopropyl-1,1,1-trifluorobut-3-yn-2-ol methanesulfonate (1:1 mol/mol, CN46630-MSA) (15.2 g, 35.6 mmol, 59% yield) was obtained as an off-white solid (99.4% purity and 99.7% *ep,* according to Method B). The combined mother liquor and wash liquor was concentrated (46.7 g residue). During storage overnight at 3 °C a white solid crystallized from the residue. The product was filtered, washed with toluene, and then toluene/isopropyl alcohol (10:1-w/w, 10g) was added. After stirring the slurry for 60 min at 30 °C the mixture was cooled to 3 °C and filtered. The product was washed with toluene/isopropyl alcohol (10:1-w/w) and *dried in vacuo* at 40 °C. CN46630-MSA (second crop, 3.8 g, 8.0 mmol, 13% yield) was obtained as off-white solid (89.7% purity at 99.7% *ep,* according to Method B).

**Example 3: (*R*)-4-(Cyclopropylethynyl)-6-phenyl-4-(trifluoromethyl)-1,4-dihydro-2*H*-3,1-benzoxazin-2-one**

(*R*)-2-(4-Aminobiphenyl-3-yl)-4-cyclopropyl-1,1,1-trifluorobut-3-yn-2-ol methanesulfonate (CN46630-MSA) of example 2 (14.7 g, 34.4 mmol) in ethyl acetate/heptanes (1:1 v/v, 27.9 g) was charged in a jacketed 150 mL-reactor with agitator and off-gas scrubber with caustic soda. After addition of aqueous $Na_2CO_3$ (12%-w/w, 32.3 g, 36.4 mmol), formation of gas during addition!) the mixture was stirred for 15 min at 15 °C. The aqueous phase was separated and discarded. Aqueous $Na_2CO_3$ (12%-w/w, 41 g, 46 mol) and ethyl acetate (20 g) were charged to the organic phase. Triphosgene (4.41 g, 14.9 mmol) was added in portions over a period of 25 min at 10 °C. The reaction mixture was stirred for 2 h at 8 °C. The mixture was diluted with ethyl acetate (45 g) and the phases were separated. The aqueous phase was discarded. The organic phase was washed with water (12 mL), dried over $MgSO_4$, filtered and concentrated and dried at 50 °C under reduced pressure to obtain (*R*)-4-(cyclopropylethynyl)-6-phenyl-4-(trifluoromethyl)-1,4-dihydro-2*H*-3,1-benzoxazin-2-one (CN46685, compound of formula IV, wherein $R^1$ is trifluoromethyl, $R^2$ is cyclopropyl, $R^8$ is 6-phenyl, $R^9$ is hydrogen and $R^{10}$ is hydrogen) (12.1 g, 33.9 mmol, 98%) as a yellowish solid (99.5% purity, according to Method C).

**Example 4: (*R*)-2-(2-Amino-5-fluorophenyl)-4-cyclopropyl-1,1,1-trifluorobut-3-yn-2-ol methanesulfonate (2:3 mol/mol)**

(1*S*,2*R*)-1-phenyl-2-(pyrrolidinyl)propan-1-ol (18.2%-w/w, 20.3 g, 18.0 mmol) in THF/toluene (9:1-w/w) was charged under nitrogen to a dry, jacketed 150 mL-reactor with agitator. Diethylzinc in toluene (29.9%-w/w, 6.20 g, 15.0 mmol) was added by syringe while keeping the temperature at 17 to 22 °C. Then the mixture was aged for 30 min at 17 °C. Cyclopropylacetylene (compound of formula III, wherein $R^2$ is cyclopropyl) in toluene (69.6%-w/w, 6.82 g, 71.8 mmol) was added at 17 °C and the reaction mixture was aged for 60 min at 20 °C. To the reaction mixture BuLi in toluene (19.3 g, 3.06 mol/kg, 59.1 mmol) and 1-(2-amino-5-fluorophenyl)-2,2,2-trifluoroethanone (CAS [214288-07-0], CN46221, compound of formula III, wherein A is 2-amino-5-fluorophenyl and $R^1$ is trifluoromethyl) (36.9%-w/w, 33.7 g, 60.0 mmol) in THF/toluene (1:1-w/w) were added in parallel over a period of 3 h at 20 °C. The addition of BuLi was kept 10 min in advance of the CN46221 addition. After completed addition the reaction mixture was stirred at 20 °C for 30 min, heated over a period of 60 min to 30 °C and aged for 6 h at 30 °C. The reaction mixture was stirred overnight at 0 °C. HPLC indicated 82.4% conversion and 96.0% *ep* of (*R*)-2-(2-amino-5-fluorophenyl)-4-cyclopropyl-1,1,1-trifluorobut-3-yn-2-ol (CN46619, formula I, wherein A is 2 amino-5 fluorophenyl, $R^1$ is trifluoromethyl and $R^2$ is cyclopropyl) according to Method B. The reaction mixture was diluted with toluene (27.6 g) and quenched by adding aqueous citric acid (1 M, 45.3 g). The phases were separated and the aqueous phase was discarded. The organic phase was successively washed with water (9.1 g), aqueous $NaHCO_3$ (5%-w/w, 24.2 g) and water (12.0 g). The organic phase was alternating concentrated and diluted with toluene to remove THF. The obtained residue (51.0 g) was diluted with isopropyl alcohol (16.7 g) and toluene (60.0 g). Methanesulfonic acid (8.36 g, 87.0 mmol) was added by a syringe pump over a period of 75 min at 30 °C. The mixture was aged and cooled stepwise over 2 h 10 min to reach 5 °C before the mixture was filtered. The filter cake was washed with toluene/isopropyl alcohol (10:1-w/w, 27.0 g) and dried under reduced pressure at 40 °C. The dry product (*R*)-2-(2-amino-5-fluorophenyl)-4-cyclopropyl-1,1,1-trifluorobut-3-yn-2-ol methanesulfonate (2:3 mol/mol, CN46619-MSA, compound of formula I, wherein A is 2-amino-5-fluorophenyl, $R^1$ is trifluoromethyl and $R^2$ is cyclopropyl) (19.46 g, 46.6 mmol, 78% yield) was obtained as a yellowish solid (99.8%-w/w by $^1H$-NMR, and 99.8% *ep,* according to Method B).

**Example 5: (*R*)-4-(Cyclopropylethynyl)-6-fluoro-4-(trifluoromethyl)-1,4-dihydro-2*H*-3,1-benzoxazin-2-one**

(*R*)-2-(2-Amino-5-fluorophenyl)-4-cyclopropyl-1,1,1-trifluorobut-3-yn-2-ol methanesulfonate (CN46619-MSA) of example 4 (14.0 g, 33.5 mmol) in ethyl acetate/heptanes (40 g, 6/4 v/v) was charged to a jacketed 150 mL-reactor

with agitator and off-gas scrubber with caustic soda. After addition of aqueous $Na_2CO_3$ (12%-w/w, 26.9 g, 30.3 mmol) the mixture was stirred for 5 min at 15 °C. The aqueous phase was separated and discarded. Aqueous $Na_2CO_3$ (12%-w/w, 34.1 g, 38.4 mmol) was charged to the organic phase, then triphosgene (3.73 g, 12.6 mmol) was added in portions over a period of 25 min at 10 °C. The reaction mixture was stirred for 2 h at 8 °C. The mixture was charged with heptanes (15.9 g), the phases were separated and the aqueous phase discarded. The organic phase was washed with water (12 mL), dried over $MgSO_4$, filtered and concentrated to dryness. After drying under vacuum at 50 °C, the product (*R*)-4-(cyclopropylethynyl)-6-fluoro-4-(trifluoromethyl)-1,4-dihydro-2*H*-3,1-benzoxazin-2-one (CN46686, compound of formula IV, wherein $R^1$ is trifluoromethyl, $R^2$ is cyclopropyl, $R^8$ is 6-fluorophenyl, $R^9$ is hydrogen and $R^{10}$ is hydrogen 9.78 g, 32.7 mmol, 97%) was obtained as a yellowish solid (99.4% purity, according to Method C).

**Example 6: Cyclisation of SD573 with diphosgene**

Aqueous $Na_2CO_3$ (12%-w/w, 183 g, 0.206 mol) was charged to SD573-MSA ((*S*)-2-(2-amino-5-chlorophenyl)-4-cyclopropyl-1,1,1-trifluorobut-3-yn-2-ol mesylate (2:3 mol/mol) = methanesulfonate of the S-enantiomer of the compound of formula I, wherein A is 2-amino-5-chlorophenyl, $R^1$ is trifluoromethyl and $R^2$ is cyclopropyl; 100 g, 0.23 mol, corresponding to 66.8 g of SD573 free base, 99.6% *ee*, prepared accordingly to Example 1) in ethyl acetate/heptanes (203 g, 1.5:1 v/v). The mixture was stirred for 5 min at 15 °C. Hydrolysis of the mesylate ended at about pH 9.0 of the aqueous phase. Then a phase separation was performed and the aqueous phase was removed. Aqueous $Na_2CO_3$ (12%-w/w, 232 g, 0.262 mol) was charged to the organic phase. To the biphasic mixture, liquid diphosgene (24 g, 120 mmol) was added in 90 min at 12 °C. After a conversion of more than 99.7% was reached, according to Method C, heptanes (204 g) were charged. The reaction mixture was then heated to 20 °C, the aqueous phase was separated and discarded, while the organic phase was washed with water (about 80 g). The organic layer was heated under reduced pressure, ethyl acetate distilled off and heptanes were charged to the reaction mixture to achieve a residual ethyl acetate content of 5.5%-w/w and a ratio of heptanes to organic matter of 10 L/kg in view of originally added SD573-MSA. Then the mixture was heated to dissolve all organic matter. The solution was seeded with 0.8 g of DMP-266 (DMP-266 = *S*-enantiomer of the compound of formula IV, wherein $R^1$ is trifluoromethyl, $R^2$ is cyclopropyl, $R^8$ is 6-chloro, $R^9$ is hydrogen and $R^{10}$ is hydrogen) at 55 °C and stirred for about 15 min at 55 °C. The mixture was then cooled to 50 °C and hold for 120 min. Then the mixture was further cooled within 2 h from 50 °C to 25 °C and within another 2 h ramp to about -10 °C. Finally, the mixture was stirred for about 1 h at -10 °C maximum and then filtered. The filter cake (wet product) was washed with pre-cooled heptanes (2×50 mL) at 0 °C maximum. The solid was dried *in vacuo* to yield 94.2% (68.4 g, 216 mmol) of DMP-266 of formula IV, wherein $R^1$ is trifluoromethyl, $R^2$ is cyclopropyl, $R^8$ is 6-chloro, $R^9$ and $R^{10}$ are hydrogen at a purity of 99.9%-w/w according to Method D. The sample comprises 99.8%-w/w *S*-enantiomer, i.e. an enantiomeric excess (*ee*) of 99.6%.

**Example 7: Cyclisation of SD573 with diphosgene**

Aqueous $Na_2CO_3$ (12%-w/w 91.5 g, 0.103 mol) was charged to SD573-MSA (50 g, 0.115 mol, corresponding to 33.4 g of SD573 free base, 99.6% *ee,* prepared accordingly to Example 1) in ethyl acetate/heptanes (101.5 g, 1.5/1 v/v). The mixture was stirred for 5 min at 15 °C. Hydrolysis of the mesylate ended at pH 6.4 in the aqueous phase. A phase separation was performed and the aqueous phase was removed. The remaining organic phase was cooled to 12 °C and aqueous $Na_2CO_3$ (12%-w/w, 106 g, 0.12 mol) was charged. To the biphasic mixture, liquid diphosgene (11.4 g, 57 mmol) was added in 90 min at 12 °C. After a total conversion was reached according to Method C, heptanes (68.8 g) was charged. The reaction mixture was then heated to 20 °C, stirred 30 min and the aqueous phase was removed. The organic phase was heated under reduced pressure, ethyl acetate was distilled off and heptanes charged to the reaction mixture to achieve a residual ethyl acetate content of 4.4 w% and a ratio of heptanes to organic matter of 6.5 L/kg in view of originally added SD573-MSA. Then the obtained mixture was heated to dissolve all organic matter. The solution was seeded with DMP-266 (0.4 g) at 55 °C and stirred for about 15 min at 55 °C. The mixture was then cooled to 50 °C and hold for 120 min. The mixture was further cooled in 2 h from 50°C to 25°C and within another 2 h to -10 °C maximum. The mixture finally was stirred for about 1 h at -13 °C and then filtered. The filter cake (wet product) was washed with pre-cooled heptanes (2×50 mL) at 0 °C maximum. The solid was dried *in vacuo* to yield 92.1% (33.45 g, 105 mmol) of DMP-266 of formula IV, wherein $R^1$ is trifluoromethyl, $R^2$ is cyclopropyl, $R^8$ is 6-chloro, $R^9$ and $R^{10}$ are hydrogen of a purity of 99.9%-w/w according to Method D. The sample comprises 99.8%-w/w *S* enantiomer, i.e. 99.6% *ee.*

**Example 8: Cyclisation of SD573 with triphosgene**

SD573-MSA (50 g, 0.114 mol, corresponding to 33.4 g of SD573 free base, 99.6% *ee*, prepared accordingly to Example 1) was dissolved in an ethyl acetate/heptanes mixture (164 g, 1:1 v/v) and charged with aqueous $Na_2CO_3$ (12%-w/w, 91.5 g, 0.104 mol). After hydrolysis of the mesylate a pH of about 7.0 was measured in the aqueous phase.

The mixture was stirred for at least 5 min at 15 °C. Then a phase separation was performed and the aqueous phase was removed. The mixture was cooled below 12 °C and aqueous $Na_2CO_3$ (12%-w/w, 116 g, 0.131 mol) was charged. To the biphasic mixture, triphosgene (12.5 g, 42 mmol) was added at 10 °C maximum in five portions within 90 min. The mixture was stirred further 15 min below 15 °C. After a total conversion was reached according to Method C, heptanes (54 g) was charged. The reaction mixture was then heated to 20 °C and the aqueous phase was removed. The organic phase was washed with water (40 g) and then heated under reduced pressure, ethyl acetate distilled off and heptanes charged to the reaction mixture to achieve a residual ethyl acetate content of 4.6%-w/w and a ratio of heptanes to organic matter of 6.5 L/kg in view of originally added SD573-MSA. The solution was seeded with DMP 266 (0.4 g) at 57 °C and stepwise cooled under stirring at -10 °C within 2 h 15 min. The mixture was stirred at -10 °C maximum overnight and then filtered. The filter cake was washed with pre-cooled heptanes (2×25 mL) at 0 °C maximum. The solid was dried in vacuo to yield 95% of DMP-266 of formula IV, wherein $R^1$ is trifluoromethyl, $R^2$ is cyclopropyl, $R^8$ is 6-chloro, $R^9$ and $R^{10}$ are hydrogen (34.22 g, 108 mmol) at a purity of 100%-w/w, according to Method D. The sample comprises 99.8%-w/w *S*-enantiomer, i.e. 99.6% *ee.*

**Example 9: Cyclisation of SD573 with triphosgene**

SD573-MSA (100 g, 0.23 mol, corresponding to 66.8 g of SD573 free base, 99.6% *ee,* prepared accordingly to Example 1) was dissolved in ethyl acetate/heptanes (203 g, 1.5:1 v/v) and charged with aqueous $Na_2CO_3$ (12%-w/w, 183 g, 0.207 mol) at about 15 °C. A pH of 7 to 9 was reached in the aqueous phase. The mixture was stirred for 5 min at 15 °C. Then the phase separation was performed and the aqueous phase was removed. The mixture was cooled below 12 °C and aqueous $Na_2CO_3$ (12%-w/w, 232 g, 0.263 mol) was charged. To the biphasic mixture, triphosgene (24.08 g, 81 mmol) was added in 10 portions within 120 min at less than 12 °C. The mixture was stirred further 10 min at about 12°C. After a total conversion was reached according to Method C, heptanes (204 g) were charged. The reaction mixture was then heated to 20 °C and the aqueous phase was removed. The organic phase was washed with water (80 g) and then heated under reduced pressure to partially remove ethyl acetate, while heptanes were charged to the reaction mixture to achieve a residual ethyl acetate content of 5.8%-w/w and a ratio of heptanes to organic matter of 7.0 L/kg in view of originally added SD573-MSA. The solution was seeded with DMP-266 (0.8 g) at 55 °C and stirred for 15 min. Then the mixture was cooled to 50 °C within 20 min, hold for 2 h, cooled to 25 °C within 2 h, cooled to about -10 °C within 2 h. After cooling to about -10 °C and stirring overnight the mixture was filtered. The isolated product was washed with pre-cooled heptanes (2×50 mL) at -10 °C maximum. The solid was dried *in vacuo* to yield 85.4% DMP-266 of formula IV, wherein $R^1$ is trifluoromethyl, $R^2$ is cyclopropyl, $R^8$ is 6-chloro, $R^9$ and $R^{10}$ are hydrogen (62 g, 19.6 mmol) at a purity of 100%-w/w, according to Method D. The sample comprises 99.8%-w/w *S*-enantiomer, i.e. 99.6% *ee.*

**Example 10: Cyclisation of SD573 with triphosgene**

Aqueous $Na_2CO_3$ (14%-w/w, 135 g, 0.178 mol) was charged to SD573 free base (33.4 g, 0.115 mol) in ethyl acetate/heptanes (70.4 g of 45:55 v/v) at 15 °C. The mixture was cooled to 8 °C and triphosgene in heptanes (26.8%-w/w, 112 g, 101 mmol) was added within 60 min, while the temperature was kept at 5 °C to 12 °C. After 60 min a total conversion was reached, according to Method C. The reaction mixture was heated to 25 °C. Then a phase separation was performed and the aqueous phase was removed. The organic phase was heated under reduced pressure, ethyl acetate partly was distilled off and heptanes were charged to the reaction mixture to achieve a residual ethyl acetate content of about 2.5%-w/w and a ratio of heptanes to organic matter of about 15 L/kg in view of originally added SD573 free base. Then the mixture was heated to dissolve all organic matter and afterwards seeded with DMP-266 (overall 1.4 g) at 55 °C. No product crystallized and therefore the organic phase was heated under reduced pressure to partially remove ethyl acetate, while heptanes were charged to the reaction mixture to achieve a residual ethyl acetate content of less than 3% (w/w) and a ratio of heptanes to organic matter of about 15 L/kg in view of originally added SD573 free base. Then the mixture was heated to dissolve all organic matter and afterwards seeded with DMP-266 (1.5 g) at 51 °C and stirred for about 140 h at 51 °C. The slurry was stepwise cooled under stirring within 4 min to reach -15 °C. The slurry was stirred for 16 h at -15 °C and then filtered. The isolated product was washed with pre-cooled heptanes (2×55 mL) at -10 °C maximum. The solid was dried *in vacuo* to yield 92.9% (33.7 g, 107 mmol) of DMP-266, at a purity of 99.6%-w/w, according to Method D. The sample comprises 99.8%-w/w *S*-enantiomer, i.e. 99.6% *ee.*

**Example 11: Cyclisation of SD573 with triphosgene**

Aqueous $Na_2CO_3$ (12%-w/w, 91.5 g, 0.103 mol) was charged to SD573-MSA (50 g, 0.115 mol, corresponding to 33.4 g of SD573 free base, prepared accordingly to Example 1) in ethyl acetate/heptanes (90.8 g, 55/45 v/v). The mixture was stirred for 5 min at 15 °C resulting in a pH of 6.8 of the aqueous phase. Then a phase separation was performed and the aqueous phase was removed. The organic phase was heated under reduced pressure and the solvent

was partially removed (32.3 g, 41 mL) to obtain a ratio of SD573 free base to solvent of about 1:1.75 (w/w). The distillate contained about 53.2 w% of ethyl acetate. The mixture comprising the SD573 free base was cooled to 12 °C and aqueous $Na_2CO_3$ (12%-w/w, 96 g, 0.109 mol) was charged. To the biphasic mixture, triphosgene in ethyl acetate (31%-w/w, 32.7 g, 34 mmol) was added in 66 min at 7 to 12 °C. The mixture was stirred 15 min at 12 °C maximum. After a conversion of 90.2% was reached, according to Method C, additional heptanes (86 g) were charged and the reaction mixture was heated to 20 °C. Then a phase separation was performed and the aqueous phase was removed. The organic phase was heated under reduced pressure to partially remove ethyl acetate while heptanes were charged to the organic phase to achieve a residual ethyl acetate content of 6.8%-w/w (target 3 to 7%-w/w) and a ratio of heptanes to organic matter of 6.8 L/kg in view of originally added SD573-MSA. The solution was seeded with DMP-266 (0.4 g) at 47 °C and stirred for 150 min at 47 to 55 °C. Then the mixture was slowly cooled to -10 °C and filtered. The filter cake was washed with pre-cooled heptanes (2×25 mL) at -10 °C maximum. The solid was *dried in vacuo* to yield 81.1% (29.46 g, 0.093 mmol) of DMP-266 of a purity of 97.2%-w/w according to Method D.

**Example 12: Cyclisation of SD573 with triphosgene**

Aqueous $Na_2CO_3$ (12%-w/w, 275.1 g, 0.311 mol) was charged to SD573-MSA (150 g, 0.345 mol, corresponding to 100.2 g of SD573 free base, prepared accordingly to Example 1) in ethyl acetate/heptanes (272.1 g, 55/45 v/v). After stirring the mixture for 5 min at 15 °C a pH of 7.7 was measured. Then a phase separation was performed and the aqueous phase was discarded. The organic phase (ethyl acetate/heptanes ratio of 61.5/38.5 w/w) was split into 3 parts each comprising about 33 g of SD573 free base. With an aim to test the stability of SD573 free base in ethyl acetate/heptanes mixtures, the 1st part was stored for 4 days at 4 °C before performing Example 12.1, the 2nd part was stored for 7 days at 4 °C before performing Example 12.2, and the 3rd part was stored for 10 days at 4 °C before performing Example 12.3.

**Example 12.1:**

The 1st part of the organic phase of Example 12 (123.5 g) was heated under reduced pressure to partially remove the solvent until the distillate contained 60 w% of ethyl acetate (about 33 g). The remaining mixture was cooled to 12 °C and aqueous $Na_2CO_3$ (12%-w/w, 117 g, 0.132 mol) was charged. To the biphasic mixture, triphosgene in ethyl acetate (36%-w/w, 35 g, 42 mmol) was added in 60 min at less than 12 °C. The mixture was stirred 15 min at less than 12 °C. After a total conversion was reached according to Method C, heptanes (86 g) were charged and the reaction mixture was heated to 20 °C. Then a phase separation was performed and the aqueous phase was removed. The organic phase was washed with water (40 g). The organic phase was heated under reduced pressure to party remove ethyl acetate, while heptanes were charged to the reaction mixture to achieve a residual ethyl acetate content of 5.5%-w/w (target 3 to 7%-w/w). A ratio of heptanes to organic matter of 6.3 L/kg in view of originally added SD573-MSA was obtained for crystallisation. The solution was seeded with DMP-266 of formula IV, wherein $R^1$ is trifluoromethyl, $R^2$ is cyclopropyl, $R^8$ is 6-chloro, $R^9$ and $R^{10}$ are hydrogen (0.4 g) at 57 °C and stirred for 15 min at the seeding temperature. The mixture was stepwise cooled under stirring to -15 °C within 6 h 20 min, stirred overnight at -10 °C and finally filtered. The filter cake was washed with pre-cooled heptanes (2x25 mL) at -10 °C maximum. The solid was dried *in vacuo* to yield 89.4% (32.17 g, 102 mmol) of DMP-266 at a purity of 100%-w/w according to Method D.

**Example 12.2:**

The 2nd part of the organic phase of Example 12 (122.0 g) was heated under reduced pressure to partially remove the solvent until the distillate contained 53 w% of ethyl acetate (about 31 g). The mixture was cooled to 12 °C and aqueous $Na_2CO_3$ (12%-w/w, 117 g, 0.132 mol) was charged. To the biphasic mixture triphosgene in ethyl acetate (36%-w/w, 35 g, 42 mmol) was added in 60 min at 12 °C maximum. The mixture was stirred for 15 min at 12 °C maximum. A total conversion was obtained according to Method C. Heptanes (86 g) were charged and the reaction mixture was heated to 20 °C. Then a phase separation was performed and the aqueous phase was removed. The organic phase was washed with water (40 g) and then heated under reduced pressure to partially remove ethyl acetate, while heptanes were charged to the reaction mixture to achieve a residual ethyl acetate content of 5.7%-w/w (target 3 to 7%-w/w). A ratio of heptanes to organic matter of 6.4 L/kg in view of originally added SD573-MSA was obtained for crystallisation. The mixture was seeded with DMP-266 of formula IV, wherein $R^1$ is trifluoromethyl, $R^2$ is cyclopropyl, $R^8$ is 6-chloro, $R^9$ and $R^{10}$ are hydrogen (0.4 g) at 57 °C and stepwise cooled under stirring within 6 h to reach -15 °C. Then the mixture was stirred at -10 °C overnight and filtered. The filter cake was washed with pre-cooled heptanes (2×50 mL) at -10 °C maximum. The solid was dried *in vacuo* to yield 89.5% (32.21 g, 102 mmol) of DMP-266 at a purity of 100%-w/w according to Method D.

**Example 12.3:**

The 3rd part of the organic phase of Example 12 (122.5 g) was heated under reduced pressure to partially remove the solvent until the distillate contained 53.6 w% of ethyl acetate (about 32.3 g). The mixture was cooled to 9 °C before aqueous $Na_2CO_3$ (12%-w/w, 117 g, 0.132 mol) was charged. To the biphasic mixture triphosgene in ethyl acetate (36%-w/w, 35 g, 42 mmol) was added in 60 min at 12 °C maximum and the mixture stirred for 1 h at 12 °C maximum. A total conversion was obtained according to Method C. Heptanes (86 g) were charged and the reaction mixture was heated to 20 °C. A phase separation was performed and the aqueous phase was removed. The organic phase was washed with water (40 g) and then heated under reduced pressure to partially remove ethyl acetate, while heptanes were charged to the reaction mixture to achieve a residual ethyl acetate content of 5.8%-w/w. A ratio of heptanes to organic matter of 6.2 L/kg in view of originally added SD573-MSA was obtained for crystallisation. The mixture was seeded with DMP-266 (0.4 g) at 57 °C and stepwise cooled under stirring to -15 °C within 6 h. The mixture was stirred at -10 °C overnight and then filtered. The filter cake was washed with pre-cooled heptanes (2×25 mL) at -10 °C maximum. The solid was dried *in vacuo* to yield 90% of DMP-266 of formula IV, wherein $R^1$ is trifluoromethyl, $R^2$ is cyclopropyl, $R^8$ is 6-chloro, $R^9$ and $R^{10}$ are hydrogen (32.4 g, 103 mmol) at a purity of 100%-w/w according to Method D.

**Example 13: Cyclisation of SD573 with triphosgene**

Aqueous $Na_2CO_3$ (14%-w/w, 160 g, 0.211 mol) was charged to SD573-MSA (100 g, 0.229 mol, corresponding to 66.8 g of SD573 free base, prepared accordingly to Example 1) in ethyl acetate/heptanes (158.8 g 1/1 v/v). The mixture was stirred at approx. 15 °C resulting in a pH of 6.8 of the aqueous phase. Then a phase separation was performed and the aqueous phase was removed. The organic phase was cooled to 12 °C and aqueous $Na_2CO_3$ (14%-w/w, 214 g, 0.283 mol) was charged. To the biphasic mixture triphosgene in ethyl acetate (35.7%-w/w, 67.2 g, 81 mmol) was added in 60 min at 12 °C maximum. The mixture was stirred for 30 min at 12°C maximum. Heptanes (96 g) were charged and a total conversion was obtained according to Method C. The reaction mixture was heated to 20°C. Then a phase separation was performed and the aqueous phase was removed. Aqueous $Na_2CO_3$ (14%-w/w, 92 g, 0.121 mol) was added to the organic phase and stirred for 25 min at 20 °C. Then a phase separation was performed and the aqueous phase was removed. The organic phase (360 g) was split into two parts.

**Example 13.1:**

The 1st part of the organic phase of Example 8 (180 g) was washed with water (80 g), phase separation was performed and the aqueous phase was removed. Then the organic phase was heated under reduced pressure, ethyl acetate partially distilled off and heptanes charged to the reaction mixture to achieve a residual ethyl acetate content of 3%-w/w (target 3 to 7%-w/w) was obtained. Finally total 10 L/kg SD573-MSA heptanes was achieved for the crystallisation.

The solution was seeded with DMP-266 (0.2 g) at 55 °C and stepwise cooled under stirring to -15 °C within 7 h. The mixture was stirred at -15 °C overnight and then filtered. The filter cake was washed with pre-cooled heptanes (2×50 mL) at -10 °C maximum. The solid was dried *in vacuo* to yield 93% (33.47 g, 105 mmol) of DMP-266 of formula IV, wherein $R^1$ is trifluoromethyl, $R^2$ is cyclopropyl, $R^8$ is 6-chloro, $R^9$ and $R^{10}$ are hydrogen, the purity is 98.8%-w/w, according to Method D.

**Example 13.2:**

The 2nd part of the organic phase of Example 8 (180 g) was heated under reduced pressure to partially remove ethyl acetate, while heptanes were charged to the reaction mixture, to achieve a residual ethyl acetate content of 3.4%-w/w (target 3 to 7%-w/w). A ratio of heptanes to organic matter of 10 L/kg in view of originally added SD573-MSA was obtained for crystallisation. The mixture was seeded with 0.2 g of DMP-266 at 55 °C and stepwise cooled under stirring within 6 h 40 min to reach -15 °C. The mixture was stirred at -10 °C overnight and then filtered. The filter cake was washed with pre-cooled heptanes (2×50 mL) at -10 °C. maximum. The solid was *dried in vacuo* to yield 96% (34.87 g, 110 mmol) of DMP-266 of formula IV, wherein $R^1$ is trifluoromethyl, $R^2$ is cyclopropyl, $R^8$ is 6-chloro, $R^9$ and $R^{10}$ are hydrogen at a purity of 97.7%-w/w according to Method D.

**Example 14: Cyclisation of SD573 with triphosgene**

Aqueous $Na_2CO_3$ (14%-w/w 80 g, 0.106 mol) was charged to SD573-MSA (50 g, 0.115 mol, corresponding to 33.4 g of SD573 free base, prepared accordingly to Example 1) in of ethyl acetate/heptanes (79.4 g, 1/1 v/v) and charged. After stirring for 15 min a pH of 6.4 was measured in the aqueous phase. The mixture was stirred for 5 min at 15 °C. Then a phase separation was performed and the aqueous phase was removed. The mixture was cooled to 12 °C and

aqueous $Na_2CO_3$ (14%-w/w, 107 g, 0.141 mol) was charged. Triphosgene in ethyl acetate (35.7%-w/w, 33.6 g, 40.5 mmol) was added to the biphasic mixture for 60 min at 12 °C maximum. The mixture was stirred 30 min at 12°C maximum. Heptanes (48 g) were charged and a total conversion was obtained according Method C. The reaction mixture was heated to 20 °C. Then a phase separation was performed and the aqueous phase was removed. The organic phase was washed with water (80 g) and then heated under reduced pressure, to partially remove ethyl acetate, while heptanes were charged to the reaction mixture, to achieve a residual ethyl acetate content of 3.2%-w/w. A ratio of heptanes to organic matter of 9.6 L/kg in view of originally added SD573-MSA was obtained for crystallisation. The mixture was seeded with 0.2 g of DMP-266 at 55 °C, and stepwise cooled under stirring within 7 h to reach -15 °C. The mixture was stirred at -15 °C overnight and then filtered. The filter cake was washed with pre-cooled heptanes (50 mL) at -10 °C maximum. The solid was dried *in vacuo* to yield 97% (34.49 g, 110 mmol) of DMP-266 of formula IV, wherein $R^1$ is trifluoromethyl, $R^2$ is cyclopropyl, $R^8$ is 6-chloro, $R^9$ and $R^{10}$ are hydrogen at a purity of 96.5%-w/w according to Method D.

**Example 15: Cyclisation of SD573 with triphosgene**

Aqueous $Na_2CO_3$ (14%-w/w, 80 g, 0.106 mol) was charged to SD573-MSA (50 g, 0.114 mol, corresponding to 33.4 g of SD573 free base, prepared accordingly to Example 1) in ethyl acetate/heptanes (79.4 g, 1/1 v/v). After stirring for 15 min a pH of 6.1 was measured in the aqueous phase. The mixture was stirred for 5 min at 15 °C. Then the phase separation was performed and the aqueous phase was removed. The mixture was cooled to 12 °C and aqueous $Na_2CO_3$ (14% w/w, 135 g, 0.178 mol) was charged. To the biphasic mixture triphosgene in ethyl acetate (35.7%-w/w, 33.6 g, 40.5 mmol) was added in 60 min at 12 °C maximum. The mixture was stirred 30 min at 12°C maximum. Heptanes (48 g) were charged and a total conversion was obtained according to Method C. The reaction mixture was heated to 20 °C. Then a phase separation was performed and the aqueous phase was removed. The organic phase was washed with water (80 g) and then heated under reduced pressure to partially remove ethyl acetate, while heptanes were charged to the reaction mixture, to achieve a residual ethyl acetate content of 2.8%-w/w. A ratio of heptanes to organic matter of 9.5 L/kg in view of originally added SD573-MSA was obtained for crystallisation. The solution was seeded with DMP-266 (0.2 g) at 55 °C and stepwise cooled under stirring within 4 h 35 min to reach to -15 °C. The mixture was stirred overnight at -15 °C and then filtered. The filter cake was washed with pre-cooled heptanes (2×50 mL) at -10 °C. maximum. The solid was dried *in vacuo* to yield 97.6% of DMP-266 of formula IV, wherein $R^1$ is trifluoromethyl, $R^2$ is cyclopropyl, $R^8$ is 6-chloro, $R^9$ and $R^{10}$ are hydrogen (35.12 g, 111 mmol) at a purity of 95.1%-w/w according to Method D.

**Example 16: Cyclisation of SD573 with phosgene**

Aqueous $Na_2CO_3$ (12%-w/w, 183 g, 0.207 mol) was charged to SD573-MSA (100 g, 0.228 mol, corresponding to 66.8 g af SD573 free base, prepared accordingly to Example 1) in ethyl acetate/heptanes (203 g, 1.5:1 v/v). After stirring for 15 min a pH of 7.2 was measured in the aqueous phase. The mixture was stirred for 5 min at 15 °C. Then the phase separation was performed and the aqueous phase was removed. The mixture was cooled to 12 °C and aqueous $Na_2CO_3$ (12%-w/w, 232 g, 0.263 mol) was charged. Phosgene (24.8 g, 251 mmol) was added to the biphasic mixture in 90 min at 12 °C maximum. Heptanes (136 g) were charged to the mixture and a total conversion was obtained according to Method C. The reaction mixture was heated to 20 °C. Then a phase separation was performed and the aqueous phase was removed. The organic phase was washed with water (80 g) and then heated under reduced pressure to partially remove ethyl acetate, while heptanes were charged to the reaction mixture, to achieve a residual ethyl acetate content of less than 7%-w/w. A ratio of heptanes to organic matter of 9.7 L/kg in view of originally added SD573-MSA was obtained for crystallisation. The solution was seeded with DMP-266 (0.8 g) at 55 °C, stepwise cooled under stirring within 6 h 15 min to reach -15 °C and then filtered. The filter cake was washed with pre-cooled heptanes (2×50 mL) at 0 °C maximum. The solid was dried *in vacuo* to yield 95.7% of DMP-266 of formula IV, wherein $R^1$ is trifluoromethyl, $R^2$ is cyclopropyl, $R^8$ is 6-chloro, $R^9$ and $R^{10}$ are hydrogen (68.91 g, 218 mmol) at a purity of 100%-w/w according to Method D.

**Example 17: Cyclisation of SD573 with phosgene**

SD573-MSA (50 g, 0.114 mol, corresponding to 33.4 g oF SD573 free base, prepared accordingly to Example 1) was dissolved in ethyl acetate/heptanes (102 g, 55:45 v/v) and charged with aqueous $Na_2CO_3$ (12%-w/w, 91 g, 0.103 mol). After stirring for 15 min a pH of about 7 was measured in the aqueous phase. The mixture was stirred for 5 min at 15 °C. Then the phase separation was performed and the aqueous phase was separated and discarded. The organic phase was cooled to 12 °C and charged with aqueous $Na_2CO_3$ (12%-w/w, 157 g, 0.178 mol). To the biphasic mixture phosgene (16.9 g, 171 mmol) was added in 130 min at 12 °C maximum. Heptanes (43 g) were charged and a total conversion was obtained according to Method C. The reaction mixture was heated to 20 °C. Then a phase separation was performed and the aqueous phase was removed. The organic phase was washed with water (80 g) and then heated

under reduced pressure to partially remove ethyl acetate, while heptanes were charged to the reaction mixture, to achieve a residual ethyl acetate content of 3.5%-w/w. A ratio of heptanes to organic matter of ca. 10 L/kg in view of originally added SD573-MSA was obtained for crystallisation. The solution was seeded with DMP-266 (0.4 g) at 62 °C and stepwise cooled under stirring to -5 °C overnight and then filtered. The filter cake was washed with pre-cooled heptanes (2×50 mL) at 0 °C maximum. The solid was *dried in vacuo* to yield 93% of DMP-266 of formula IV, wherein $R^1$ is trifluoromethyl, $R^2$ is cyclopropyl, $R^8$ is 6-chloro, $R^9$ and $R^{10}$ are hydrogen (33.86 g, 107 mmol) at a purity of 98.5%-w/w according to Method D.

**Example 18: (*S*)-2-(2-Amino-5-methylphenyl)-4-cyclopropyl-1,1,1-trifluorobut-3-yn-2-ol**

A solution of (1*R*,2*S*)-1-phenyl-2-(pyrrolidinyl)propan-1-ol (17.6%-w/w, 21.0 g, 18.0 mmol) in a THF/toluene mixture (9:1-w/w) was charged in a vessel at room temperature. A solution of diethylzinc in toluene (29.9%-w/w, 6.10 g, 14.8 mmol) was added at 17 to 25 °C and the mixture was aged at said temperature range for 30 min. A solution of cyclopropylacetylene (compound of formula II, wherein $R^2$ is cyclopropyl) in toluene (69.6%-w/w, 8.55 g, 90.0 mmol) was added at 18 °C and the resulting mixture was aged at 20 °C for 60 min. A solution of BuLi in toluene (3.09 mol/kg, 17.6 g, 54.4 mmol) and a solution of 1-(2-amino-5-methylphenyl)-2,2,2-trifluoroethanone (CN46217, compound of formula III, wherein A is 2-amino-5-methylphenyl and $R^1$ is trifluoromethyl) (36.5%-w/w, 33.4 g, 60.0 mmol) in toluene/THF (1:1-w/w) were added in parallel to the reaction mixture at 20 °C within 3 h. The addition of BuLi was started 10 min in advance of the CN46217 addition. After completed addition of CN46217 the reaction mixture was stirred for 30 min at 20 °C, then heated to 30 °C over a period of 60 min and aged for 6 h at 30 °C. The reaction mixture was stirred at 0 °C overnight. HPLC (Method B) indicated 72.3% conversion and 96.7% enantiomeric purity. The reaction mixture was diluted with toluene (25.8 g) and quenched by addition of aqueous citric acid (1 M, 73.9 g). After stirring for 15 min the phases were separated and the aqueous phase was discarded. The organic phase was successively washed with water (9.1 g), aqueous $NaHCO_3$ solution (5%-w/w, 24.0 g), and water (12.0 g). The organic phase was partially concentrated (60 g residual solution), diluted with toluene (30 g), and partially concentrated again (52 g residue). The residue was diluted with toluene (65 g), cooled to 5 °C and aged over night. The crystals were filtered, washed with cold (approx. 5 °C) toluene (10 g) and dried under vacuum at 40 °C. The wet product (10.8 g) obtained as off-white solid with a purity of 99.2 and 100% *ep* according to method B. The crude product was purified by slurring it in a mixture of toluene (10 mL) and heptane (40 mL) at room temperature for 1 h, filtered and dried at 40 °C *in vacuo.* The product (CN46624, compound of formula I, wherein A is 2-amino-5-methylphenyl, $R^1$ is trifluoromethyl and $R^2$ is cyclopropyl) was obtained as white solid (10.6 g, 38 mmol, 64% yield) with a purity of 99.4% and 100% *ep* according to method B. The assay was 97.0%-w/w according to $^1$H-NMR.

**Example 19: (*S*)-4-(Cyclopropylethynyl)-6-methyl-4-(trifluoromethyl)-1,4-dihydro-2*H*-3,1-benzoxazin-2-one**

(2*S*)-2-(2-Amino-5-metbylphenyl)-4-cyclopropyl-1,1,1-trifluorobut-3-yn-2-ol (CN46624) obtained according example 18 (97,0%-w/w, 10.0 g, 36.0 mmol) in ethyl acetate/heptanes (2:1-w/w, 30 g) was charged to a jacketed 150 mL-reactor with agitator and off-gas scrubber with caustic soda. The reaction mixture was cooled to 7 °C and aqueous $Na_2CO_3$ solution (12%-w/w, 33.5 g) was added. Triphosgene (3.67 g, 12.4 mmol) was added in portions over a period of 25 min at 7 to 15 °C. The reaction mixture was stirred for 15 min at 8 °C and sampled for conversion control (99.8% conversion according to method C). The precipitated solid was dissolved by adding ethyl acetate (25 g) and the phases were separated. The organic phase was washed with water (10 g), dried over $MgSO_4$, filtered and concentrated under vacuum to dryness. The crude product (11.6 g) was obtained as a white solid (purity 96.9% according to HPLC method C). Hexane (20 mL) was added and the mixture was stirred for 1 h at room temperature. The product was filtered, washed with cold hexane (10 mL) and dried at 35 °C under vacuum. The product (compound of formula IV, wherein $R^1$ is trifluoromethyl, $R^2$ is cyclopropyl, $R^8$ is 6-methyl, $R^9$ is hydrogen and $R^{10}$ is hydrogen) was obtained as white solid (9.76 g, 32.9 mmol, 91 % yield) with a purity of 98.8% according to method C and an assay of 99.6%w-/w according to $^1$H-NMR.

**Example 20: 2-(2-Amino-5-chlorophenyl)-1,1,1-trifluorooct-3-yn-2-ol methanesulfonate (2:3 mol/mol)**

**Example 20.1: (*R*)-2-(2-Amino-5-chlorophenyl)-1,1,1-trifluorooct-3-yn-2-ol methanesulfonate (2:3 mol/mol)**

A solution of (1*S*,2*R*)-1-phenyl-2-(pyrrolidinyl)propan-1-ol (18.7%-w/w, 19.7 g, 18.0 mmol) in THF/toluene (9:1-w/w) was charged to a vessel at room temperature. A solution of diethylzinc in toluene (29.9%-w/w, 6.10 g, 14.8 mmol) was added at 17 to 25 °C and the mixture was aged at said temperature for 30 min, 1-hexyne (97%-w/w, 6.10 g, 72.0 mmol, compound of formula II, wherein $R^2$ is *n*-butyl) was added at 18 °C and the resulting solution was aged at 20 °C for 60 min. A solution of BuLi in toluene (3.09 mol/kg, 17.8 g, 55.0 mmol) and a solution of 1-(2-amino-5-chlorophenyl)-2,2,2-trifluoroethanone (CN23315 or SD570, a compound of formula III, wherein A is 2-amino-5-chlorophenyl, $R^1$ is

trifluoromethyl and) in toluene/THF (1:1 w/w) (39.6%-w/w, 33.8 g, 60.0 mmol) were added in parallel to the reaction mixture at 20 °C within 3 h. The addition of BuLi was started 10 min in advance of the CN23315 addition. After completed addition of CN23315 the reaction mixture was stirred for 30 min at 20 °C, then heated to 30 °C over a period of 60 min and aged for 6 h at 30 °C. The reaction mixture was stirred at 0 °C overnight. HPLC (Method B) indicated 89.6% conversion. The reaction mixture was diluted with toluene (25.8 g) and quenched by addition of aqueous citric acid solution (1 M, 44.1 g). After stirring for 15 min the phases were separated and the organic phase successively washed with water (9.1 g), aqueous $NaHCO_3$ solution (5%-w/w, 24.0 g) and water (12.0 g). The organic phase was partially concentrated (51 g residual solution), diluted with toluene (30 g), and partially concentrated again (58 g residue). The residue was diluted with toluene (59 g) and isopropyl alcohol (1.50 g). Methanesulfonic acid (10.48 g, 114 mmol) was added at 30 °C over a period of 30 min and the mixture was stirred for 30 main. A second portion methanesulfonic acid (2.89 g, 30 mmol) was added at 30 °C over a period of 30 min. The mixture was stirred at 30 °C for 30 min, cooled to 5 °C over a period of 60 min, and aged at 5 °C for 30 min. The crystals were filtered, washed with cold toluene (10 g) and dried under vacuum at 40 °C. The crude product (19.3 g) was obtained as yellowish solid with a purity of 93.3% and 99.6% *ep* according to method B. The product was further purified by slurring it in a mixture of toluene (100 mL) and isopropyl alcohol (2 mL) at room temperature for 3 h. The product (MSA salt of (R)-CN47583, compound of formula I, wherein A is 2-amino-5-chlorophenyl, $R^1$ is trifluoromethyl and $R^2$ is *n*-butyl) was filtered, washed with toluene (10 mL) and dried at 40 °C under vacuum. The product was obtained as white solid (17.1 g, 35.3 mmol, 59% yield) with a purity of 93.3% and 99.9% *ep* according to method B, and an assay of 92.8%w-/w according to $^1$H-NMR.

**Example 20.2: (*S*)-2-(2-Amino-5-chlorophenyl)-1,1,1-trifluorooct-3-yn-2-ol methanesulfonate (2:3 mol/mol)**

Example 20.1 was repeated with (1*R*,2*S*)-1-phenyl-2-(pyrrolidinyl)propan-1-ol as chiral ligand to obtain the (*S*)-enantiomer of CN47583.

A solution of (1*R*,2S)-1-phenyl-2-(pyrrolidinyl)propan-1.-ol (17.6%-w/w, 42.0 g, 36.0 mmol) in THF/toluene (9:1-w/w) was charged a vessel at room temperature. A solution of diethylzinc in toluene (29.9%-w/w, 12.0 g, 29.05 mmol) was added at 17 to 25 °C and the mixture was aged at said temperature for 30 min, 1-hexyne (97%-w/w, 13.21 g, 156.0 mmol, compound of formula II, wherein $R^2$ is *n*-butyl) was added at 18°C and the resulting solution was aged at 20 °C for 60 min. A solution of BuLi in toluene (3.09 mol/kg, 35.53 g, 109.8 mmol) and a solution of 1-(2-amino-5-chlorophenyl)-2,2,2-trifluoroethanone (CN23315 or SD570, a compound of formula III, wherein A is 2-amino-5-chlorophenyl, $R^1$ is trifluoromethyl and) in toluene/THF (1:1 w/w) (39.6%-w/w, 67.75 g, 120.0 mmol) were added in parallel to the reaction mixture at 20 °C within 3 h. The addition of BuLi was started 10 min in advance of the CN23315 addition. After completed addition of CN23315 the reaction mixture was stirred for 30 min at 20 °C, then heated to 30 °C over a period of 60 min and aged for 6 h at 30 °C. The reaction mixture was stirred at 0 °C overnight. HPLC (Method B) indicated 81.9% conversion. The reaction mixture was diluted with toluene (51.6 g) and quenched by addition of aqueous citric acid solution (1 M, 88.2 g). After stirring for 15 min the phases were separated and the organic phase successively washed with water (18.1 g), aqueous $NaHCO_3$ solution (5%-w/w, 48.0 g) and water (24.0 g). The organic phase was partially concentrated (110 g residual solution), diluted with toluene (60 g), and partially concentrated again (114 g residue). The residue was diluted with toluene (120 g). Isopropyl alcohol (3.2 g) was added. Methanesulfonic acid (10.96 g, 114 mmol) was added at 30 °C over a period of 30 min and the mixture was stirred for 30 min. A second portion methanesulfonic acid (5.78 g, 60 mmol) was added at 30 °C over a period of 30 min. The mixture was stirred at 30 °C for 30 min, cooled to 5 °C over a period of 60 min, and aged at 5 °C for 30 min. The crystals were filtered, washed with cold toluene/isopropyl alcohol (98:1, 1×25 mL, 2×120 mL) and dried under vacuum at 40 °C. The product (MSA salt of compound of formula I, wherein A is 2-amino-5-chlorophenyl, $R^1$ is trifluoromethyl and $R^2$ is *n*-butyl, 28.57 g) was obtained as slightly beige solid (96.5%w-/w assay according to $^1$H-NMR).

**Example 21: (*R*)-6-Chloro-4-(hex-1-yn-1-yl)-4-(trifluoromethyl)-1,4-dihydro-2*H*-3,1-benzoxazin-2-one**

(*R*)-2-(2-Amino-5-chlorophenyl)-1,1, -trifluorooct-3-yn-2-ol methanesulfonate ((*R*)-CN47583) obtained according to example 20.1 (92.8%-w/w as methanesulfonate 2:3 mol/mol, 15.0 g, 30.9 mmol) in ethyl acetate/heptanes (2:1-w/w, 30 g) was charged to a jacketed 150 mL-reactor with agitator and off-gas scrubber with caustic soda. The reaction mixture was cooled to 15 °C and aqueous $Na_2CO_3$ solution (12%-w/w, 27 g, formation of gas during addition!) was added, and then the mixture was stirred for 5 min at 15 °C. The aqueous phase was separated and removed. Aqueous $Na2CO_3$ solution (12%-w/w, 33 g) was added to the organic phase. Triphosgene (3.62 g, 12.2 mmol) was added in portions over a period of 25 min at 7 to 15 °C. The reaction mixture was stirred for 15 min at 8 °C and sampled for conversion control (conversion more than 99% according to method C). The phases were separated. The organic phase was dried over $MgSO_4$ filtered and concentrated under vacuum to dryness. The crude product (11.4 g) was obtained as yellow oil (purity more than 99.0 % according to method C). A sample was cooled to 5 °C and it slowly solidified. The crude product was slurried in hexane (10 mL)) for 2 h at room temperature. The product was filtered, washed with cold

(approx. 5°C) hexane (5 mL) and dried at 30 °C under vacuum. The product ((*R*)-compound of formula IV, wherein $R^1$ is trifluoromethyl, $R^2$ is *n*-butyl, $R^8$ is 6-chloro, $R^9$ is hydrogen and $R^{10}$ is hydrogen) was obtained as white solid (7.73 g, 22.7 mmol, 73% yield) with a purity of more than 99.0% according to method C and an assay of 97.1%-w/w according to $^1$H-NMR. Concentration of the mother liquor to dryness under vacuum afforded additional product as yellow solid (2.54 g, 7.2 mmol, 23% yield) with a purity of 98% according to method C and an assay of 93.6%-w/w according to $^1$H-NMR.

**Example 22: (*S*)-6-Claloro-4-(hex-1-yn-1-yl)-4-(trifluoromethyl)-1,4-dihydro-2*H*-3,1-benzoxazin-2-one**

(*S*)-2-(2-Amino-5-chlorophenyl)-1 ,1,1-trifluorooct-3-yn-2-ol methanesulfonate ((*S*)-CN47583) obtained according to example 20.2 (96.5%-w/w as methanesulfonate 2:3 mol/mol, 15.0 g, 32.2 mmol) in ethyl acetate/heptanes (2:1-w/w, 30 g) was charged to a jacketed 150 mL-reactor with agitator and off-gas scrubber with caustic soda. The reaction mixture was cooled to 15 °C and aqueous $Na_2CO_3$ solution (12%-w/w, 27 g, formation of gas during addition!) was added, and then the mixture was stirred for 5 min at 15 °C. The aqueous phase was removed. And aqueous $Na_2CO_3$ solution (12%-w/w, 33 g) was added to the organic phase. A solution of triphosgene (0.73 g, 2.5 mmol) in diphosgene (2.90 g, 14.7 mmol) was added to the reaction mixture over a period of 30 min at 7 to 11 °C. The reaction mixture was stirred at 8 °C for 20 min. The reaction mixture was sampled for conversion control until a conversion of more than 99% was reached (according to method C). The phases were allowed to separate. The aqueous phase was removed. The organic phase was dried over $MgSO_4$, filtered and concentrated to dryness. The crude product (10.5 g, 31.1 mmol, 97% yield) was obtained as yellow solid (purity >99.0%, HPLC method C, 98.6%-w/w assay by $^1$H-NMR). The crude product was slurried in hexane (10 mL) for 3 h at room temperature. The product was filtered, washed with cold (approx. 5 °C) hexane (5 mL) and dried at 30 °C under vacuum. The product ((S)-compound of formula IV, wherein $R^1$ is trifluoromethyl, $R^2$ is *n*-butyl, $R^8$ is 6-chloro, $R^9$ is hydrogen and $R^{10}$ is hydrogen) was obtained as white solid (8.25 g, 24.6 mmol, 77% yield) with a purity of more than 99.0% (according to method C) and assay 99.1%-w/w according to $^1$H-NMR. Concentration of the mother liquor to dryness under vacuum afforded additional product as yellow solid (1.58 g) with a purity of 97% (according to method C).

**Example 23: Cyclisation of SD573 (free base) with diphosgene in triphosgene**

Triphosgene (5.12 g, 17 mmol) was added to diphosgene (20.16 g, 101 mmol) at 8 °C and the mixture was aged under rigorous stirring for 30 min (until all triphosgene was dissolved).

In another vessel, aqueous $Na_2CO_3$ (12%-w/w, 235 g, 266 mmol) was charged at 8 °C to SD573 free base (compound of formula I, wherein A is 2-amino-5-chlorophenyl, $R^1$ is trifluoromethyl and $R^2$ is cyclopropyl, 67.0 g, 0.231 mol) in heptane (68.3 g) and ethyl acetate (136.1 g). Then the solution of triphosgene in diphosgene was added at 8 to 11 °C within 90 min. The mixture was aged further 45 min at 8 °C. The mixture was warmed to 15 °C within ca. 30 min and aged further 30 min at 15 °C, total conversion was reached according to Method C. Heptane (137 g) was added at 15 °C and the mixture was aged for further 60 min at 15 °C. The mixture was warmed to 19 °C and water (80 g) was added. The phases were separated and the aqueous phase was removed. The organic phase was distilled and heptane continuously added until 5.4-w/w% of ethyl acetate remained (concentration of the heptane solution was approx. 9.5 mL/g of SD573). The mixture was seeded at 58 °C with DMP-266 (0.8 g) and the suspension was stirred further 120 min at 58 °C, cooled to 25 °C within 120 min, cooled to -13 °C within 120 min, stirred further ca. 30 min at -13 °C and filtered. The wet cake was washed at -8 °C two times with heptane (pre-cooled at -8°C, 50 mL). The cake was dried for 8 h at 80 °C under vacuum. 90.2% yield (65.99 g, 209 mmol) of product (DMP-266, compound of formula IV, wherein $R^1$ is trifluoromethyl, $R^2$ is cyclopropyl, $R^8$ is 6-chloro, $R^9$ is hydrogen and $R^{10}$ is hydrogen) were obtained with a purity of 100%-w/w according to Method D. Crystal form I was obtained according to X-ray analysis.

**Analytical Methods:**

**Method A:** (HPLC method used for the determination of the enantiomeric purity)

Column: Chiralpak® AD, 250x4.6 mm; Temperature: 40°C; Flow: 1.0 mL/min; Mobile Phase: hexane/isopropyl alcohol = 75:25 (v/v); UV Detection: 260 nm

**Method B:** (HPLC method used for conversion, purity and enantiomeric purity):

Column: Chiralpak® AD-H, 250×4.6 mm; Temperature: 40 °C; Flow: 1.0 mL/min; Mobile phase: hexane/isopropyl alcohol = 89:11 (v/v); UV Detection: 260 nm

**Method C:** (HPLC method used for the determination of the purity):

Column: Zorbax® RX-C18, 250×4.6 mm, 5 micrometer; Temperature: 40°C; Flow: 1.5 mL/min; Mobile phase A: 50 %-w/w buffer / 50 %-w/w MeCN; Mobile phase B: MeCN; Buffer: 0.1 %-w/w $H_3PO_4$ in water, pH adjusted to 3.6; Gradient: 0 min 0 %-w/w B to 30 min 90 %-w/w B; UV Detection: 250 nm

**Method D:** (HPLC method used for the determination of the purity):

Column: Zorbax® SB-CN, 150×4.6 mm; Temperature: 40°C; Flow: 1.5 mL/min; Mobile phase A: 90 %-w/w water/10 %-w/w MeOH + 0.05 %-w/w TFA (v/v); Mobile phase B: 90% water/10 %-w/w MeOH + 0.05 %-w/w TFA (v/v); Gradient: 16 min 40 %-w/w to 50% B, 7 min to 65 %-w/w B, 5 min to 70% B, 1 min to 80% of B, 2 min hold 80 %-w/w B, 1 min to 40 %-w/w B; UV Detection: 250 nm

## Claims

**1.** A process for the preparation of a compound of formula

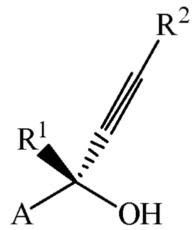

I,

or mirror image, wherein

$R^1$ is selected from the group consisting of hydrogen, $C_{1-6}$-alkyl and ($C_{1-6}$-alkoxy)carbonyl, any alkyl or alkoxy optionally being substituted with one or more halogen atoms,
$R^2$ is selected from the group consisting of aryl, aralkyl, $C_{1-6}$-alkyl and (1'-$R^3$)-$C_{3-6}$-cycloalkyl wherein $R^3$ is hydrogen, methyl or ethyl, and wherein any aryl, aralkyl, alkyl is optionally substituted with one or more halogen atoms, and
A is selected from the group consisting of $C_{1-20}$-alkyl, $C_{3-6}$-cycloalkyl, aryl and aralkyl, any cycloalkyl, aryl and aralkyl optionally being annulated to one or more further 5 to 7 membered carbocyclic or heterocyclic rings, and wherein any alkyl, cycloalkyl, aryl and aralkyl is optionally substituted with one or more substituents selected from halogen atoms, cyano, $C_{1-6}$-alkyl, $C_{3-6}$-cycloalkyl, -$NR^4R^5$, -$SR^6$, S(O) $R^6$ or S($O_2$)$R^6$, and/or -$OR^7$, with $R^6$ is $C_{1-6}$-alkyl, optionally substituted with one or more halogen atoms,
$R^7$ is hydrogen or $C_{1-6}$-alkyl, optionally substituted with one or more halogen atoms, where

(a) $R^4$ and $R^5$ are independently selected from hydrogen or $C_{1-6}$-alkyl, or
(b) $R^4$ is hydrogen and $R^5$ is $C_{2-7}$-acyl or ($C_{1-6}$-alkoxy)carbonyl, wherein each acyl and alkoxy in $R^5$ in turn is optionally substituted with one or more halogen atoms, or
(c) $R^4$ and $R^5$ together with the nitrogen atom form a 5 to 7 membered heterocyclic ring, or
(d) $R^4$ and $R^5$ together are =CH-aryl, the aryl moiety optionally being substituted with one or more substituents selected from halogen atoms, -$NH_2$, -NH($C_{1-6}$-alkyl), -N($C_{1-6}$-alkyl)$_2$ or $C_{1-6}$-alkyl, or
(e) $R^4$ and $R^5$ together are =CH-N($C_{1-6}$-alkyl)$_2$,

$R^6$ is $C_{1-6}$-alkyl, optionally substituted with one or more halogen atoms, and
$R^7$ is hydrogen or $C_{1-6}$-alkyl, optionally substituted with one or more halogen atoms, or, wherein A and $R^1$ together form a 5 to 7 membered carbocyclic or heterocyclic ring, optionally substituted with one or more substituents selected from halogen atoms, cyano, $C_{1-6}$-alkyl, $C_{3-6}$-cycloalkyl, -$NR^4R^5$, -$SR^6$, S(O) $R^6$ or S($O_2$)$R^6$, and/or -$OR^7$, wherein $R^2$, $R^3$, $R^4$, $R^5$, $R^6$ and $R^7$ are as defined above, and wherein each alkyl and cycloalkyl substituent attached to A in turn is optionally substituted with one or more halogen atom,
said process comprising the steps of

(i) reacting a protic chiral auxiliary, wherein said protic chiral auxiliary is an ephedrine derivative selected

from the group consisting of diastereoisomers of 2-(di-$C_{1-4}$-alkylamino)-1-phenyl-propan-1-ols, 2-(*N,N*-$C_{4-6}$-alkylene)-1-phenyl-propan-1-ols and 2-(1-heteroaryl)-1-phenyl-propan-1-ols"
with a diorganylzinc(II) compound selected from the group consisting of di($C_{1-18}$-alkyl)zinc(II) and di($C_{3-6}$-cycloalkyl)zinc(II),
in the presence of a solvent selected from the group consisting of tetrahydrofuran, benzene, chlorobenzene, *o-, m-, p*-dichlorobenzene, dichloromethane, toluene, *o*-, *m-,* and *p*-xylene, hexanes, heptanes, pentane, 1,4-dioxane, cyclohexane, diethyl ether, *tert*-butyl methyl ether, diisopropyl ether, *N*-methylpyrrolidine, and mixtures thereof, at a temperature in the range of 0 to 40 °C, and
(ii) keeping the mixture of step (i), preferably under stirring, in a first maturation period until the reaction is completed, but of at least 20 min, and
(iii) reacting the mixture obtained after step (ii) with a compound of formula

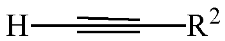 II,

wherein $R^2$ is as defined above, and
(iv) keeping the mixture of step (iii), preferably under stirring, in a second maturation period until the reaction is completed, but of at least 10 min, and
(v) reacting the mixture obtained after step (iv) with a compound of formula

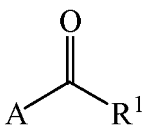 III,

wherein A and $R^1$ are as defined above, and an organolithium base selected from the group consisting of ($C_{1-6}$-alkyl)lithium, lithium diisopropylamide (LDA), lithium hexamethyldisilazide (LiHMDS), phenyllithium, and naphthyllithium,
and/or another alkali metal compound selected from sodium or potassium $C_{1-6}$-alkoxides, sodium or potassium diisopropylamide, and sodium or potassium hexamethyldisilazide, at a temperature in the range of 0 to 40 °C, and
(vi) keeping the mixture obtained in step (v) to 10 to 50 °C until the reaction is completed, to obtain the compound of formula I.

**2.** The process of claim 1, wherein the molar ratio of the protic chiral auxiliary to the diorganylzinc(II) compound is in the range of 1.5:1 to 1:1.

**3.** The process of claims 1 or 2, wherein the diorganylzinc(II) compound is a di($C_{1-8}$-alkyl)zinc(II) compound, the alkyl moieties of which are selected from the group consisting of methyl, ethyl, propyl, isopropyl, butyl, isobutyl, *sec*-butyl and *tert*-butyl, pentyl, hexyl, heptyl, and octyl

**4.** The process of claims 1 or 2, wherein the diorganylzinc(II) compound is a di($C_{3-6}$-cycloalkyl)zinc(II) compound, the cycloalkyl moieties of which are selected from the group consisting of cyclopropyl, cyclobutyl, cyclopentyl and cyclohexyl.

**5.** The process of any of claims 1 to 4, wherein in step (i) the molar ratio of the protic chiral auxiliary to the compound of formula III is in the range of 1:1 to 1:10, preferably in the range of 1:2 to 1:6, more preferably of 1:3 to 1:6.

**6.** The process of any of claims 1 to 5, wherein in step (iii) the compound of formula II is used in a molar ratio to the compound of formula III of 1:0.6 to 1:1.3.

**7.** The process of any of claims 1 to 6, wherein the organolithium base and/or the other alkali metal compound selected from sodium or potassium $C_{1-6}$-alkoxides, sodium or potassium diisopropylamide, and sodium or potassium hexamethyldisilazide is added in a molar ratio to the compound of formula III from 1:0.8 to 1:1.5.

8. The process of claim 7, wherein the ($C_{1-6}$-alkyl)lithium is selected from the group consisting of methyllithium, *n*-butyllithium, *sec*-butyllithium, *tert*-butyllithium, and hexyllithium.

9. The process of any of claims 1 to 8, wherein the temperature during the addition of the organolithium base and/or the other alkali metal compound selected from sodium or potassium $C_{1-6}$- alkoxides, sodium or potassium diisopropylamide, and sodium or potassium hexamethyldisilazide is of from +10 to +30 °C.

10. The process of any of claims 1 to 9, wherein the ephedrine derivative is a diasteromer of 2-(dimethylamino)-1-phenyl-propan-1-ol, 2-(diethylamino)-1-phenyl-propan-1-ol, 2-(diisopropylamino)-1-phenyl-propan-1-ol, 2-(dibutylamino)-1-phenyl-propan-1-ol; 1-phenyl-2-(piperidinyl)propan-1-ol, 1-phenyl-2-(pyrrolidinyl)propan-1-ol, 1-phenyl-2-(1-pyridinyl)propan-1-ol or 1-phenyl-2-(1-piridinyl)propan-1-ol.

11. The process of any of claims 1 to 9, wherein the ephedrine derivative is (1*R*,2*S*)-2-(dimethylamino)-1-phenyl-propan-1-ol, (1*S*,2*R*)-2-(dimethylamino)-1-phenyl-propan-1-ol, (1*R*,2*R*)-2-(dimethylamino)-1-phenyl-propan-1-ol, (1*S*,2*S*)-2-(dimethylamino)-1-phenyl-propan-1-ol, (1*R*,2*S*)-1-phenyl-2-(pyrrolidinyl)propan-1-ol, (1*S*,2*R*)-1-phenyl-2-(pyrrolidinyl)propan-1-ol or (1*R*,2*R*)-1-phenyl-2-(pyrrolidinyl)propan-1-ol and (1*S*,2*S*)-1-phenyl-2-(pyrrolidinyl)propan-1-ol.

**Patentansprüche**

1. Verfahren für die Herstellung einer Verbindung der Formel

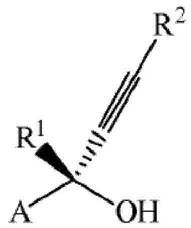

I,

oder eines Spiegelbilds davon, wobei $R^1$ ausgewählt ist aus Wasserstoff, $C_{1-6}$-Alkyl und ($C_{1-6}$-Alkoxy)carbonyl, wobei etwaiges Alkyl oder Alkoxy wahlweise substituiert ist mit einem oder mehreren Halogenatomen,
$R^2$ ausgewählt ist aus Aryl, Aralkyl, $C_{1-6}$-Alkyl und (1'-$R^3$)-$C_{3-6}$-Cycloalkyl, wobei $R^3$ Wasserstoff, Methyl oder Ethyl ist, und wobei etwaiges Aryl, Aralkyl, Alkyl wahlweise substituiert ist mit einem oder mehreren Halogenatomen und
A ausgewählt ist aus $C_{1-20}$-Alkyl, $C_{3-6}$-Cycloalkyl, Aryl und Aralkyl, wobei etwaiges Cycloalkyl, Aryl und Aralkyl wahlweise anneliert sind zu einem oder mehreren weiteren 5- bis 7-gliedrigen carbocyclischen oder heterocyclischen Ringen, und wobei etwaiges Alkyl, Cycloalkyl, Aryl und Aralkyl wahlweise substituiert ist mit einem oder mehreren Substituenten, ausgewählt aus Halogenatomen, Cyano, $C_{1-6}$-Alkyl, $C_{3-6}$-Cycloalkyl, $-NR^4R^5$, $-SR^6$, S(O) $R^6$ oder $S(O_2)R^6$ und/oder $-OR^7$, wobei $R^6$ $C_{1-6}$-Alkyl ist, wahlweise substituiert mit einem oder mehreren Halogenatomen,
$R^7$ Wasserstoff oder $C_{1-6}$-Alkyl ist, wahlweise substituiert mit einem oder mehreren Halogenatomen, wobei

(a) $R^4$ und $R^5$ unabhängig ausgewählt sind aus Wasserstoff oder $C_{1-6}$-Alkyl, oder
(b) $R^4$ Wasserstoff ist und $R^5$ $C_{2-7}$-Acyl oder ($C_{1-6}$-Alkoxy)carbonyl ist, wobei jedes Acyl und Alkoxy in $R^5$ wiederum wahlweise substituiert ist mit einem oder mehreren Halogenatomen, oder
(c) $R^4$ und $R^5$ bilden zusammen mit dem Stickstoffatom einen 5- bis 7-gliedrigen heterocyclischen Ring, oder
(d) $R^4$ und $R^5$ sind zusammen =CH-Aryl, wobei der Aryl-Teil wahlweise substituiert ist mit einem oder mehreren Substituenten, ausgewählt aus Halogenatomen, $-NH_2$, -NH($C_{1-6}$-Alkyl), -N($C_{1-6}$-Alkyl$)_2$ oder $C_{1-6}$-Alkyl, oder
(e) $R^4$ und $R^5$ sind zusammen =CH-N($C_{1-6}$-Alkyl$)_2$,

$R^6$ ist $C_{1-6}$-Alkyl, wahlweise substituiert mit einem oder mehreren Halogenatomen, und
$R^7$ ist Wasserstoff oder $C_{1-6}$-Alkyl, wahlweise substituiert mit einem oder mehreren Halogenatomen, oder wobei A und $R^1$ zusammen einen 5- bis 7-gliedrigen carbocyclischen oder heterocyclischen Ring bilden, der wahlweise substituiert ist mit einem oder mehreren Substituenten, ausgewählt aus Halogenatomen, Cyano, $C_{1-6}$-Alkyl, $C_{3-6}$-Cycloalkyl, $-NR^4R^5$, $-SR^6$, S(O) $R^6$ oder $S(O_2)R^6$ und/oder $-OR^7$, wobei $R^2$, $R^3$, $R^4$, $R^5$, $R^6$ und

$R^7$ wie vorstehend festgelegt sind und wobei jeder an A befindliche Alkyl- und Cycloalkyl-Substituent wiederum wahlweise substituiert ist mit einem oder mehreren Halogenatomen, wobei das Verfahren die Schritte umfasst:

(i) Umsetzen eines protischen chiralen Hilfsmittels, wobei das protische chirale Hilfsmittel ein Ephedrin-Derivat ist, ausgewählt aus Diastereoisomeren von 2-(Di-$C_{1-4}$-alkylamino)-1-phenylpropan-1-ols, 2-(*N,N*-$C_{4-6}$-Alkylen)-1-phenylpropan-1-ols und 2-(1-Heteroaryl)-1-phenylpropan-1-ols, mit einer Diorganylzink(II)-Verbindung, ausgewählt aus Di($C_{1-8}$-alkyl)zink(II) und Di($C_{3-6}$-cycloalkyl)zink(II), in Gegenwart eines Lösemittels, ausgewählt aus Tetrahydrofuran, Benzol, Chlorbenzol, *o-, m-, p*-Dichlorbenzol, Dichlormethan, Toluol, *o-, m-, und p*-Xylol, Hexanen, Heptanen, Pentan, 1,4-Dioxan, Cyclohexan, Diethylether, *tert*-Butylmethylether, Diisopropylether, N-Methylpyrrolidin und Mischungen davon bei einer Temperatur im Bereich von 0 bis 40 °C, und
(ii) Halten der Mischung von Schritt (i) vorzugsweise unter Rühren für eine erste Reifephase, bis die Reaktion abgeschlossen ist, mindestens jedoch für 20 Minuten, und
(iii) Umsetzen der in Schritt (ii) erhaltenen Mischung mit einer Verbindung der Formel

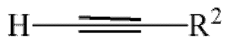

II,

wobei $R^2$ wie vorstehend festgelegt ist, und
(iv) Halten der Mischung von Schritt (iii) vorzugsweise unter Rühren für eine zweite Reifephase, bis die Reaktion abgeschlossen ist, mindestens jedoch für 10 Minuten, und
(v) Umsetzen der nach dem Schritt (iv) erhaltenen Mischung mit einer Verbindung der Formel

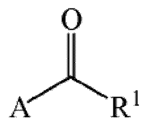

III,

wobei A und $R^1$ wie vorstehend festgelegt sind, und einer Organolithium-Base, ausgewählt aus ($C_{1-6}$-Alkyl)lithium, Lithium-Diisopropylamid (LDA), Lithium-Hexamethyldisilazid (LiHMDS), Phenyllithium und Naphthyllithium und/oder einer anderen Alkalimetallverbindung, ausgewählt aus Natrium- oder Kalium-$C_{1-6}$-alkoxiden, Natrium- oder Kalium-diisopropylamid und Natrium- oder Kalium-hexamethyldisilazid, bei einer Temperatur im Bereich von 0 bis 40 °C, und
(vi) Halten der in Schritt (v) erhaltenen Mischung bei 10 bis 50 °C, bis die Reaktion abgeschlossen ist, um die Verbindung der Formel I zu erhalten.

**2.** Verfahren nach Anspruch 1, wobei das Molverhältnis des protischen chiralen Hilfsmittels zu der Diorganylzink(II)-Verbindung im Bereich von 1,5:1 bis 1:1 liegt.

**3.** Verfahren nach Anspruch 1 oder 2, wobei die Diorganylzink(II)-Verbindung eine Di($C_1$-$_8$-alkyl)zink(11)-Verbindung ist, deren Alkyl-Teile ausgewählt sind aus Methyl, Ethyl, Propyl, Isopropyl, Butyl, Isobutyl, *sec*-Butyl und *tert*-Butyl, Pentyl, Hexyl, Heptyl und Octyl.

**4.** Verfahren nach Anspruch 1 oder 2, wobei die Diorganylzink(II)-Verbindung eine Di($C_{3-6}$-cycloalkyl)zink(II)-Verbindung ist, deren Cycloalkyl-Teile ausgewählt sind aus Cyclopropyl, Cyclobutyl, Cyclopentyl und Cyclohexyl.

**5.** Verfahren nach einem der Ansprüche 1 bis 4, wobei in Schritt (i) das Molverhältnis des protischen chiralen Hilfsmittels zu der Verbindung der Formel III im Bereich von 1:1 bis 1:10, vorzugsweise im Bereich von 1:2 bis 1:6, mehr bevorzugt von 1:3 bis 1:6 liegt.

**6.** Verfahren nach einem der Ansprüche 1 bis 5, wobei in Schritt (iii) die Verbindung der Formel II zu der Verbindung der Formel III in einem Molverhältnis von 1:0,6 bis 1:1,3 verwendet wird.

**7.** Verfahren nach einem der Ansprüche 1 bis 6, wobei die Organolithium-Base und/oder die andere Alkalimetallverbindung, die ausgewählt sind aus Natrium- oder Kalium-$C_{1-6}$-alkoxiden, Natrium- oder Kalium-diisopropylamid und Natrium- oder Kalium-hexamethyldisilazid, in einem Molverhältnis zu der Verbindung der Formel III von 1:0,8 bis 1:1,5 zugesetzt werden.

**8.** Verfahren nach Anspruch 7, wobei das ($C_{1-6}$-Alkyl)lithium ausgewählt ist aus Methyllithium, *n*-Butyllithium, *sec*-Butyllithium, *tert*-Butyllithium und Hexyllithium.

**9.** Verfahren nach einem der Ansprüche 1 bis 8, wobei die Temperatur während der Zugabe der Organolithium-Base und/oder der anderen Alkalimetallverbindung, die ausgewählt ist aus Natrium- oder Kalium-$C_{1-6}$-alkoxiden, Natrium- oder Kalium-diisopropylamid und Natrium- oder Kalium-hexamethyldisilazid, +10 bis +30 °C beträgt.

**10.** Verfahren nach einem der Ansprüche 1 bis 9, wobei das Ephedrin-Derivat ein Diasteromer von 2-(Dimethylamino)-1-phenylpropan-1-ol, 2-(Diethylamino)-1-phenylpropan-1-ol, 2-(Diisopropylamino)-1-phenylpropan-1-ol, 2-(Dibutylamino)-1-phenylpropan-1-ol; 1-Phenyl-2-(piperidinyl)propan-1-ol, 1-Phenyl-2-(pyrrolidinyl)propan-1-ol, 1-Phenyl-2-(1-pyridinyl)propan-1-ol oder 1-Phenyl-2-(1-piridinyl)propan-1-ol ist.

**11.** Verfahren nach einem der Ansprüche 1 bis 9, wobei das Ephedrin-Derivat (1*R*,2*S*)-2-(Di-methylamino)-1-phenylpropan-1-ol, (1*S*,2*R*)-2-(Dimethylamino)-1-phenylpropan-1-ol, (1*R*,2*R*)-2-(Dimethylamino)-1-phenylpropan-1-ol, (1*S*,2*S*)-2-(Dimethylamino)-1-phenylpropan-1-ol, (1*R*,2*S*)-1-Phenyl-2-(pyrrolidinyl)propan-1-ol, (1*S*,2*R*)-1-Phenyl-2-(pyrrolidinyl)propan-1-ol oder (1*R*,2*R*)-1-Phenyl-2-(pyrrolidinyl)propan-l-ol und (1*S*,2*S*)-1-Phenyl-2-(pyrrolidinyl)propan-1-ol ist.

## Revendications

**1.** Procédé pour la préparation d'un composé de formule

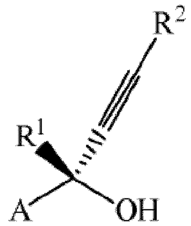

I,

ou d'une image miroir, dans lequel

$R^1$ est choisi dans le groupe constitué par hydrogène, $C_{1-6}$-alkyle et ($C_{1-6}$-alcoxy)-carbonyle, tout alkyle ou alcoxy étant facultativement substitué par un ou plusieurs atomes d'halogène,
$R^2$ est choisi dans le groupe constitué par aryle, aralkyle, $C_{1-6}$-alkyle et (1'-$R^3$)-$C_{3-6}$-cycloalkyle où $R^3$ est hydrogène, méthyle ou éthyle, et où tout aryle, aralkyle, alkyle est facultativement substitué par un ou plusieurs atomes d'halogène, et
A est choisi dans le groupe constitué par $C_{1-20}$-alkyle, $C_{3-6}$-cycloalkyle, aryle et aralkyle, tout cycloalkyle, aryle et aralkyle étant facultativement cyclisé à un ou plusieurs autres cycles carbocycliques ou hétérocycliques à 5 à 7 chaînons, et où tout alkyle, cycloalkyle, aryle et aralkyle est facultativement substitué par un ou plusieurs substituants choisis parmi les atomes d'halogène, cyano, $C_{1-6}$-alkyle, $C_{3-6}$-cycloalkyle, -$NR^4R^5$, -$SR^6$, S(O) $R^6$ ou $S(O_2)R^6$, et/ou -$OR^7$, où $R^6$ est $C_{1-6}$-alkyle, facultativement substitué par un ou plusieurs atomes d'halogène,
$R^7$ est hydrogène ou $C_{1-6}$-alkyle, facultativement substitué par un ou plusieurs atomes d'halogène, où

(a) $R^4$ et $R^5$ sont indépendamment choisis parmi hydrogène ou $C_{1-6}$-alkyle, ou
(b) $R^4$ est hydrogène et $R^5$ est $C_{2-7}$-acyle ou ($C_{1-6}$-alcoxy)carbonyle, où chaque acyle et alcoxy dans $R^5$ à leur tour sont facultativement substitués par un ou plusieurs atomes d'halogène, ou
(c) $R^4$ et $R^5$ conjointement avec l'atome d'azote forment un cycle hétérocyclique à 5 à 7 chaînons, ou
(d) $R^4$ et $R^5$ ensemble sont =CH-aryle, le groupement aryle étant facultativement substitué par un ou plusieurs substituants choisis parmi les atomes d'halogène, -$NH_2$, -NH($C_{1-6}$-alkyle), -N($C_{1-6}$-alkyle)$_2$ ou $C_{1-6}$-alkyle, ou
(e) $R^4$ et $R^5$ ensemble sont =CH-N($C_{1-6}$-alkyle)$_2$,

$R^6$ est $C_{1-6}$-alkyle, facultativement substitué par un ou plusieurs atomes d'halogène, et
$R^7$ est hydrogène ou $C_{1-6}$-alkyle, facultativement substitué par un ou plusieurs atomes d'halogène, ou
dans lequel A et $R^1$ ensemble forment un cycle carbocyclique ou hétérocyclique à 5 à 7 chaînons, facultativement

substitué par un ou plusieurs substituants choisis parmi les atomes d'halogène, cyano, $C_{1-6}$-alkyle, $C_{3-6}$-cycloalkyle, $-NR^4R^5$, $-SR^6$, S(O) $R^6$ ou $S(O2)R^6$, et/ou $-OR^7$, où $R^2$, $R^3$, $R^4$, $R^5$, $R^6$ et $R^7$ sont tels que définis ci-dessus, et où chaque substituant alkyle et cycloalkyle attaché à A à son tour est facultativement substitué par un ou plusieurs atomes d'halogène,
ledit procédé comprenant les étapes consistant

(i) à faire réagir un auxiliaire chiral protique, lequel auxiliaire chiral protique est un dérivé d'éphédrine choisi dans le groupe constitué par les diastéréomères de 2-(di-$C_{1-4}$-alkylamino)-1-phénylpropan-1-ols, 2-*(N,N*-$C_{4-6}$-alkylène)-1-phénylpropan-1-ols et 2-(1-hétéroaryl)-1-phénylpropan-1-ols,
avec un composé de diorganylzinc (II) choisi dans le groupe constitué par un di($C_{1-8}$-alkyl)zinc(II) et un di($C_{3-6}$-cycloalkyl)zinc(II),
en présence d'un solvant choisi dans le groupe constitué par le tétrahydrofuranne, le benzène, le chlorobenzène, un *o-, m-, p*-dichlorobenzène, le dichlorométhane, le toluène, un *o-, m-,* et *p*-xylène, les hexanes, les heptanes, le pentane, le 1,4-dioxane, le cyclohexane, l'éther diéthylique, l'éther de *tert*-butyl méthyle, l'éther diisopropylique, *N*-méthylpyrrolidine, et les mélanges de ceux-ci, à une température dans la gamme de 0 à 40°C, et
(ii) à maintenir le mélange de l'étape (i), de préférence sous agitation, lors d'une première période de maturation jusqu'à la fin de la réaction, mais d'au moins 20 min, et
(iii) à faire réagir le mélange obtenu après l'étape (ii) avec un composé de formule

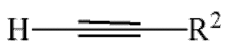
II,

où $R^2$ est tel que défini ci-dessus, et
(iv) à maintenir le mélange de l'étape (iii), de préférence sous agitation, lors d'une seconde période de maturation jusqu'à la fin de la réaction, mais d'au moins 10 min, et
(v) à faire réagir le mélange obtenu après l'étape (iv) avec un composé de formule

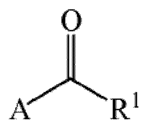
III,

où A et $R^1$ sont tels que définis ci-dessus, et une base d'organolithium choisie dans le groupe constitué par un ($C_{1-6}$-alkyl)lithium, le diisopropylamide de lithium (LDA), l'hexaméthyldisilazide de lithium (LiHMDS), le phényllithium, et le naphtyllithium, et/ou un autre composé de métal alcalin choisi parmi les $C_{1-6}$-alkoxides de sodium ou potassium, le diisopropylamide de sodium ou de potassium, et l'hexaméthyldisilazide de sodium ou de potassium, à une température dans la gamme de 0 à 40 °C, et
(vi) à maintenir le mélange obtenu dans l'étape (v) à 10 à 50 °C jusqu'à la fin de la réaction, pour obtenir le composé de formule I.

**2.** Procédé selon la revendication 1, dans lequel le rapport molaire de l'auxiliaire chiral protique au composé de diorganylzinc(II) est dans la gamme de 1.5:1 à 1:1.

**3.** Procédé selon les revendications 1 ou 2, dans lequel le composé de diorganylzinc(II) est un composé de di($C_{1-8}$-alkyl)zinc(II), dont les groupements alkyles sont choisis dans le groupe constitué par méthyle, éthyle, propyle, isopropyle, butyle, isobutyle, sec-butyle et *tert*-butyle, pentyle, hexyle, heptyle, et octyle.

**4.** Procédé selon les revendications 1 ou 2, dans lequel le composé de diorganylzinc(II) est un composé di($C_{3-6}$-cycloalkyl)zinc(II), dont les groupements cycloalkyles sont choisis dans le groupe constitué par cyclopropyle, cyclobutyle, cyclopentyle et cyclohexyle.

**5.** Procédé selon l'une quelconque des revendications 1 to 4, dans lequel dans l'étape (i) le rapport molaire de l'auxiliaire chiral protique au composé de formule III est dans la gamme de 1:1 à 1:10, de préférence dans la gamme de 1:2 à 1:6, de préférence encore de 1:3 à 1:6.

**6.** Procédé selon l'une quelconque des revendications 1 to 5, dans lequel dans l'étape (iii) le composé de formule II

est utilisé en un rapport molaire au composé de formule III de 1:0,6 à 1:1,3.

**7.** Procédé selon l'une quelconque des revendications 1 to 6, dans lequel la base d'organolithium et/ou l'autre composé de métal alcalin choisi parmi les $C_{1-6}$-alkoxides de sodium ou de potassium, le diisopropylamide de sodium ou de potassium, et l'hexaméthyldisilazide de sodium ou de potassium est ajouté en un rapport molaire au composé de formule III de 1:0,8 à 1:1,5.

**8.** Procédé selon la revendication 7, dans lequel le ($C_{1-6}$-alkyl)lithium est choisi dans le groupe constitué par le méthyllithium, le *n*-butyllithium, le *sec*-butyllithium, le *tert*-butyllithium, et l'hexyllithium.

**9.** Procédé selon l'une quelconque des revendications 1 to 8, dans lequel la température pendant l'addition de la base d'organolithium et/ou de l'autre composé de métal alcalin choisi parmi les $C_{1-6}$-alkoxides de sodium ou de potassium, le diisopropylamide de sodium ou de potassium, et l'hexaméthyldisilazide de sodium ou de potassium est de +10 à +30 °C.

**10.** Procédé selon l'une quelconque des revendications 1 à 9, dans lequel le dérivé d'éphédrine est un diastéréomère de 2-(diméthylamino)-1-phénylpropan-1-ol, 2-(diéthylamino)-1-phénylpropan-1-ol, 2-(diisopropylamino)-1-phénylpropan-1-ol, 2-(dibutylamino)-1-phénylpropan-1-ol; de 1-phényl-2-(pipéridinyl)propan-1-ol, 1-phényl-2-(pyrrolidinyl)propan-1-ol, 1-phényl-2-(1-pyridinyl)propan-1-ol ou 1-phényl-2-(1-piridinyl)propan-1-ol.

**11.** Procédé selon l'une quelconque des revendications 1 à 9, dans lequel le dérivé d'éphédrine est le (1*R*,2*S*)-2-(diméthylamino)-1-phénylpropan-1-ol, (1*S*,2*R*)-2-(diméthylamino)-1-phénylpropan-1-ol, (1*R*,2*R*)-2-(diméthylamino)-1-phénylpropan-1-ol, (1*S*,2*S*)-2-(diméthylamino)-1-phénylpropan-1-ol, (1*R*,2*S*)-1-phényl-2-(pyrrolidinyl)propan-1-ol, (1*S*,2*R*)-1-phényl-2-(pyrrolidinyl)propan-1-ol ou le *(1R,2R)*-1-phényl-2-(pyrrolidinyl)propan-1-ol et le (1*S*,2*S*)-1-phényl-2-(pyrrolidinyl)propan-1-ol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9520389 A
- WO 9637457 A
- WO 9830543 A
- WO 9830540 A
- WO 9851676 A


### Non-patent literature cited in the description


- **JIANG et al.** *Tetrahedron Lett.,* 2002, vol. 43, 8323-8325
- *J. Org. Chem.,* 2002, vol. 67, 9449-9451
- **LIU et al.** *Tetrahedron Asymmetry,* 2004, vol. 15, 3757-3761